# EUROPEAN PATENT SPECIFICATION

(11) **EP 4 131 548 B1**
(45) Date of publication and mention of the grant of the patent: **27.08.2025**
(21) Application number: 21781744.4
(22) Date of filing: 30.03.2021
(51) Int. Cl.: H01M 10/0567, C07C 311/48, C07F 5/04, C07F 9/54, C07F 9/6574, C07F 19/00, H01M 4/505, H01M 4/525, H01M 10/052, H01M 10/058, C07C 309/06, C07F 5/02, C07F 9/6571, C07F 9/06, H01M 10/0568, H01M 10/42, H01M 10/0569, H01M 10/0525

(54) **NON-AQUEOUS ELECTROLYTE FOR BATTERY, PRECURSOR FOR LITHIUM SECONDARY BATTERY, METHOD FOR MANUFACTURING LITHIUM SECONDARY BATTERY, LITHIUM SECONDARY BATTERY, PHOSPHAZENE COMPOUND, AND ADDITIVE FOR BATTERY**
WASSERFREIER ELEKTROLYT FÜR EINE BATTERIE, VORLÄUFER FÜR EINE LITHIUMSEKUNDÄRBATTERIE, VERFAHREN ZUR HERSTELLUNG EINER LITHIUMSEKUNDÄRBATTERIE, LITHIUMSEKUNDÄRBATTERIE, PHOSPHAZENVERBINDUNG UND ADDITIV FÜR EINE BATTERIE
ÉLECTROLYTE NON AQUEUX POUR BATTERIE, PRÉCURSEUR POUR BATTERIE SECONDAIRE AU LITHIUM, PROCÉDÉ DE FABRICATION DE BATTERIE SECONDAIRE AU LITHIUM, BATTERIE SECONDAIRE AU LITHIUM, COMPOSÉ PHOSPHAZÈNE ET ADDITIF POUR BATTERIE

(30) Priority: 31.03.2020 JP 2020064371
(43) Date of publication of application: 08.02.2023
(73) Proprietor: Mitsui Chemicals, Inc., Tokyo 104-0028 (JP)
(72) Inventor: MIZUNO, Yu, Sodegaura-shi, Chiba 299-0265 (JP); SUGIHARA, Yuri, Sodegaura-shi, Chiba 299-0265 (JP); HAYASHI, Takaomi, Sodegaura-shi, Chiba 299-0265 (JP); TANAKA, Emiri, Sodegaura-shi, Chiba 299-0265 (JP)
(74) Representative: J A Kemp LLP
(86) International application number: PCT/JP2021/013723
(87) International publication number: WO 2021/201054

(56) References cited:
- WO-A1-2018/025810
- WO-A1-2018/045294
- WO-A2-2012/087414
- CN-A- 108 239 265
- JP-A- 2002 003 427
- JP-A- 2004 107 266
- JP-A- 2006 036 709
- JP-A- 2008 031 348
- JP-A- 2014 501 434
- JP-A- 2017 120 370
- JP-A- H1 077 289
- US-A1- 2017 184 964
- US-A1- 2018 081 267

## Description

### Technical Field

The present disclosure relates to: a non-aqueous electrolyte solution for a battery; a lithium secondary battery precursor; a method of manufacturing a lithium secondary battery; a lithium secondary battery; a phosphazene compound; and a battery additive.

### Background Art

In order to improve the performance of a battery (e.g., a lithium secondary battery) in which a non-aqueous electrolyte solution is used, various additives are incorporated into the non-aqueous electrolyte solution.

For example, as a high-flame-retardancy electrolyte solution for non-aqueous batteries, Patent Document 1 discloses an electrolyte solution for non-aqueous batteries that contains additives including a cyclic phosphazene compound represented by (NPR₂)ₙ (wherein, each of Rs independently represents a halogen element, an alkoxy group, or an aryloxy group, and n represents 3 or 4) in a non-aqueous solvent.

Further, as a non-aqueous electrolyte battery that has no risk of rupture, ignition or the like even in the event of abnormality such as a short circuit and exhibits excellent battery performance, Patent Document 2 discloses a non-aqueous electrolyte battery including a positive electrode, a negative electrode capable of occluding and releasing lithium, and a lithium ion-containing non-aqueous electrolyte, in which a solution obtained by dissolving a lithium salt in a phosphazene derivative having a viscosity of 300 cP or less at 25°C is used as the electrolyte.
Patent Document 3 describes a lithium-free, anion based charge transport electrochemical system that uses fluoride ion transporting electrolytes, including ionic liquids, with and without various additives.
Patent Document 1: Japanese Patent Application Laid-Open (JP-A) No. 2009-129541
Patent Document 2: JP-A No. H6-13108
Patent Document 3: WO 2012/087414

### SUMMARY OF INVENTION

### Technical Problem

An object of one aspect of the disclosure is to provide: a non-aqueous electrolyte solution for a lithium secondary battery, which can inhibit an increase in the rate of temperature increase inside a battery in the event of an internal short circuit; a lithium secondary battery precursor; a method of manufacturing a lithium secondary battery; a phosphazene compound; and a lithium secondary battery additive.

### Solution to Problem

Means for solving the above-described problem include the following aspects.
<1> A non-aqueous electrolyte solution for a lithium secondary battery, the solution comprising a phosphazene compound (A) that contains at least one of a phosphazene compound represented by the following Formula (1) or a phosphazene compound represented by the following Formula (2): 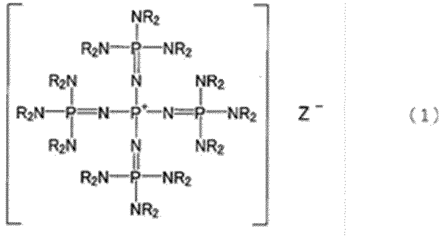 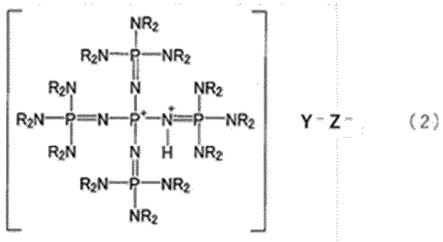 wherein, in Formula (1), Z⁻ represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other, and
   in Formula (2), each of Y⁻ and Z⁻ independently represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other 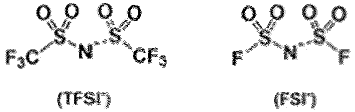 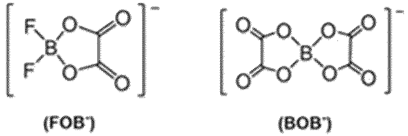 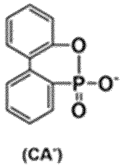
<2> The non-aqueous electrolyte solution for a lithium secondary battery according to <1>, wherein
   in Formula (1), each of the twenty-four Rs independently represents an alkyl group having from 1 to 3 carbon atoms and, among the twenty-four Rs, two Rs bonded to the same nitrogen atom are optionally bonded to each other, and
   in Formula (2), each of the twenty-four Rs independently represents an alkyl group having from 1 to 3 carbon atoms and, among the twenty-four Rs, two Rs bonded to the same nitrogen atom are optionally bonded to each other.
<3> The non-aqueous electrolyte solution for a lithium secondary battery according to <1>, wherein
   in Formula (1), each of the twenty four Rs is a methyl group or an ethyl group and, among the twenty-four Rs, two Rs bonded to the same nitrogen atom are optionally bonded to each other, and
   in Formula (2), each of the twenty four Rs is a methyl group or an ethyl group and, among the twenty-four Rs, two Rs bonded to the same nitrogen atom are optionally bonded to each other.
<4> The non-aqueous electrolyte solution for a lithium secondary battery according to <1>, wherein a content of the phosphazene compound (A) is from 0.1% by mass to 2.0% by mass with respect to a total amount of the non-aqueous electrolyte solution for a battery.
<5> The non-aqueous electrolyte solution for a lithium secondary battery according to any one of <1> to <4>, further containing a cyclic carbonic acid ester having an unsaturated bond.
<6> The non-aqueous electrolyte solution for a lithium secondary battery according to <5>, wherein the cyclic carbonic acid ester having an unsaturated bond is vinylene carbonate.
<7> The non-aqueous electrolyte solution for a lithium secondary battery according to <5> or <6>, wherein a ratio of the mass content of the cyclic carbonic acid ester having an unsaturated bond with respect to the mass content of the phosphazene compound (A) is in a range of from 0.05 to 30.
<8> A lithium secondary battery precursor, including:
   a casing; and
   a positive electrode, a negative electrode, a separator, and an electrolyte solution, which are housed in the casing,
   wherein
   the positive electrode is configured to occlude and to release lithium ions,
   the negative electrode is configured to occlude and to release lithium ions, and
   the electrolyte solution comprises the non-aqueous electrolyte solution for a lithium secondary battery according to any one of <1> to <7>.
<9> The lithium secondary battery precursor according to <8>, wherein the positive electrode contains a lithium-containing composite oxide represented by the following general formula (C1) as a positive electrode active material:

   LiNiₐCo_{b}Mn_{c}O₂ (C1)

   wherein, in the general formula (C1), each of a, b, and c independently represents a number larger than 0 but smaller than 1, and a sum of a, b, and c is from 0.99 to 1.00.
<10> A method of manufacturing a lithium secondary battery, the method including:
   preparing the lithium secondary battery precursor according to <8> or <9>; and
   performing an activation treatment of the lithium secondary battery precursor to obtain a lithium secondary battery,
   wherein the activation treatment includes performing at least one of charging or discharging of the lithium secondary battery precursor in an environment of from 15°C to 70°C.
<11> A lithium secondary battery, obtained by charging and discharging the lithium secondary battery precursor according to <8> or <9>.
<12> A phosphazene compound, containing at least one of a phosphazene compound represented by the following Formula (1) or a phosphazene compound represented by the following Formula (2): 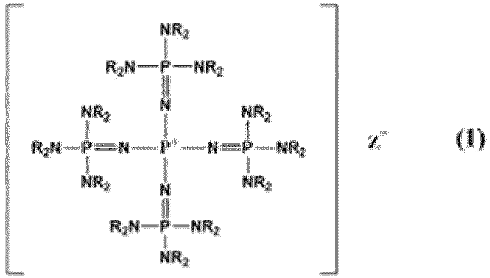 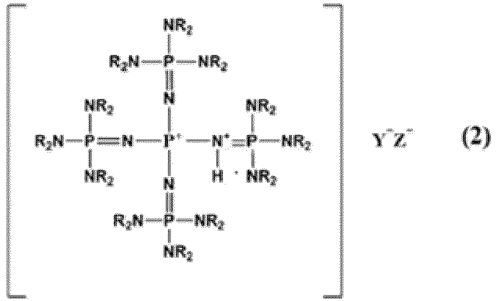
   wherein in Formula (1), Z⁻ represents PO₂F₂⁻; each of twenty-four Rs independently represents a hydrocarbon group having from 1 to 10 carbon atoms; and, among the twenty-four Rs, two Rs bonded to the same nitrogen atom are optionally bonded to each other, and
   in Formula (2), each of Y⁻ and Z⁻ independently represents an anion in which a proton is removed from an inorganic acid or an active hydrogen compound; each of twenty-four Rs independently represents a hydrocarbon group having from 1 to 10 carbon atoms; and, among the twenty-four Rs, two Rs bonded to the same nitrogen atom are optionally bonded to each other.
<13> A lithium secondary battery additive, containing a phosphazene compound (A) that contains at least one of a phosphazene compound represented by the following Formula (1) or a phosphazene compound represented by the following Formula (2): 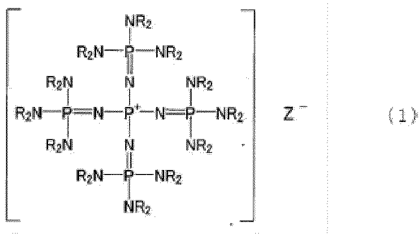 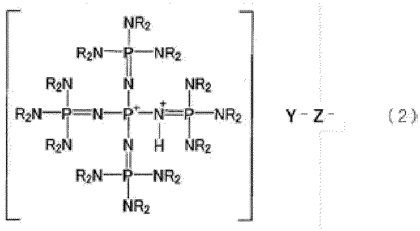
   wherein, in Formula (1), Z⁻ represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other, and
   in Formula (2), each of Y⁻ and Z⁻ independently represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other

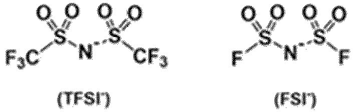

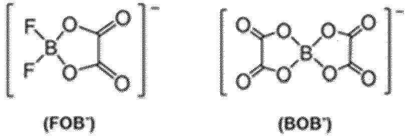

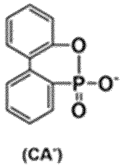

### Advantageous Effects of Invention

According to one aspect of the disclosure, the followings are provided: a non-aqueous electrolyte solution for a lithium secondary battery, which can inhibit an increase in the rate of temperature increase inside a battery in the event of an internal short circuit; a lithium secondary battery precursor; a method of manufacturing a lithium secondary battery; a phosphazene compound; and a lithium secondary battery additive.

### BRIEF DESCRIPTION OF DRAWINGS

FIG. 1 is a schematic cross-sectional view that illustrates one example of a lithium secondary battery precursor according to one embodiment of the disclosure, and
FIG. 2 is a ³¹P-NMR measurement chart of white dry solids obtained in the synthesis of tetrakis[tris(dimethylamino)phosphoranylideneamino]phosphonium difluorophosphate in one Example.

### DESCRIPTION OF EMBODIMENTS

In the present specification, those numerical ranges that are expressed with "to" each denote a range that includes the numerical values stated before and after "to" as the lower limit value and the upper limit value, respectively.

In the present specification, when there are plural substances that correspond to a component of a composition, the indicated amount of the component in the composition means, unless otherwise specified, a total amount of the plural substances existing in the composition.

In the present specification, the term "step" encompasses not only a discrete step but also a step that cannot be clearly distinguished from other steps, as long as the intended purpose of the step is achieved.

### [Phosphazene Compound (A)]

The phosphazene compound (A) contained in the non-aqueous electrolyte solution for a lithium secondary battery according to the disclosure contains at least one of a phosphazene compound represented by the following Formula (1) or a phosphazene compound represented by the following Formula (2):

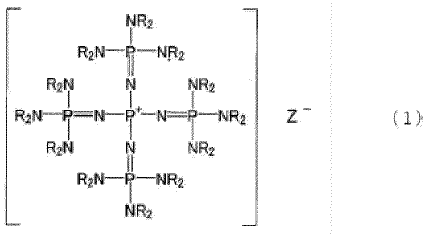

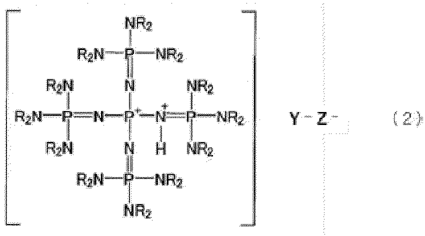

In Formula (1), Z⁻ represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other, and
in Formula (2), each of Y⁻ and Z⁻ independently represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other

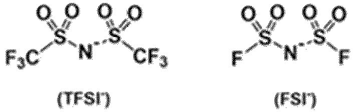

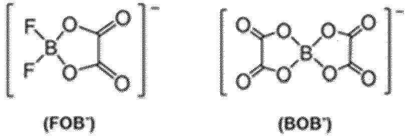

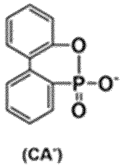

Specific examples of the inorganic acid described herein include hydrogen halides (e.g., hydrogen fluoride, hydrogen chloride, hydrogen bromide, hydrogen iodide, and bromoform), sulfuric acid, perchloric acid, phosphoric acid compounds (e.g., hexafluorophosphoric acid), boric acid compounds (e.g., tetrafluoroboric acid), hydrogen cyanide, thiocyanic acid, and hydrogen azide.

The active hydrogen compound described herein is not limited as long as it can yield an anion excluding a proton, and the active hydrogen compound may be, for example, a compound having an active hydrogen atom bonded to a carbon atom, a compound having an active hydrogen atom bonded to an oxygen atom, a compound having an active hydrogen atom bonded to a nitrogen atom, or a compound having an active hydrogen atom bonded to a sulfur atom.

Examples of the compound having an active hydrogen atom bonded to a carbon atom as described herein include compounds in which, for example, a cyano group, a nitro group, a phenyl group, an acyl group, an alkoxycarbonyl group, an alkenyl group, or an alkynyl group is bonded to a carbon atom having at least one hydrogen atom, and compounds in which an active hydrogen atom is directly bonded to an alkenyl group or an alkynyl group.

Specific examples of the compound having an active hydrogen atom bonded to a carbon atom as described herein include: cyano group-containing compounds, such as acetonitrile, *n*-valeronitrile, *n*-butyronitrile, adiponitrile, malononitrile, phenyl acetonitrile, succinonitrile, 3-methoxypropionitrile, 4-methylbenzyl cyanide, 2-nitrophenylacetonitrile, 4-methoxyphenylacetonitrile, glutaronitrile, 3-phenylpropionitrile, cyclopentenylacetonitrile, isopropylidenemalononitrile, benzoylacetonitrile, and pivaloylacetonitrile; alkoxycarbonyl group-containing compounds, such as methyl acetate, *n*-propyl acetate, isopropyl acetate, 2-methoxyethyl acetate, *tert*-butyl acetate, phenyl acetate, ethyl *n*-butyrate, ethyl propionate, benzyl acetate, *n*-amyl acetate, acetic anhydride, ethyl isovalerate, methyl levulinate, and 2-methylcyclohexyl acetate; nitro group-containing compounds, such as nitroethane, 1-nitropropane, 1-nitrobutane, methyl 4-nitrobutyrate, methyl nitroacetate, nitrocyclopentane, dinitromethane, and 1,1-dinitroethane; acyl group-containing compounds such as 2-butanone, 2-hexanone, 2-heptanone, 4,4-dimethyl-2-pentanone, methoxyacetone, cyclohexyl methyl ketone, acetophenone, benzylacetone, acetylacetone, 1-benzoylacetone, 1,3-cyclopentanedione, 1,3-cyclohexanedione, dibenzoylmethane, anthrone, 1,3-indanedione, and 3,5-heptanedione; alkenyl group-containing compounds, such as 2,4-dimethyl-2-pentene, 2-methyl-1-phenylpropene, 1-methyl-1-cyclopentene, 6,6-dimethylfulvene, 1,2,3,4,5-pentamethylcyclopentadiene, and 1,3,5,5-tetramethyl-1,3-cyclohexadiene; alkynyl group-containing compounds, such as 1-butyne, 2-butyne, 1-phenyl-1-propyne, 2-pentyne, methyl propargyl ether, 4-methyl-2-pentyne, 2,4-hexadiyne, 2-butynyl acetate, and acetylene; phenyl group-containing compounds, such as diphenylmethane, triphenylmethane, xanthene, 9,10-dihydroanthracene, fluorene, 2,7-dinitrofluorene, 4,4'-difluorodiphenylmethane, 4-benzylbiphenyl, 4-nitrodiphenylmethane, and 4,4'-dinitrodiphenylmethane; malonic acid esters, such as dimethyl malonate, diethyl malonate, di-n-butyl malonate, di-*tert*-butyl malonate, and benzylmethyl malonate; acetoacetic acid esters, such as methyl acetoacetate, ethyl acetoacetate, *n*-butyl acetoacetate, and sec-butyl acetoacetate; and cyanoacetic acid esters, such as methyl cyanoacetate, ethyl cyanoacetate, *n*-butyl cyanoacetate, isobutyl cyanoacetate, *tert*-butyl cyanoacetate, 2-ethylhexyl cyanoacetate, and benzyl cyanoacetate.

Examples of the compound having an active hydrogen atom bonded to an oxygen atom as described herein include: water; carboxylic acids having a total of from 1 to 20 carbon atoms including a carbon atom(s) of a carbonyl group(s); carbamic acids having a total of from 2 to 20 carbon atoms including a carbon atom(s) of a carbonyl group(s); sulfonic acids having from 1 to 20 carbon atoms; alcohols having from 1 to 20 carbon atoms; sugars and derivatives thereof; hydroxy group-containing aromatic compounds having from 6 to 20 carbon atoms; and polyalkylene oxides.

Examples of the carboxylic acids having a total of from 1 to 20 carbon atoms including a carbon atom(s) of a carbonyl group(s) as described herein include: monocarboxylic acids, such as formic acid, acetic acid, butyric acid, isobutyric acid, lauric acid, stearic acid, oleic acid, phenylacetic acid, dihydrocinnamic acid, cyclohexanecarboxylic acid, benzoic acid, and 2-carboxynaphthalene; and polycarboxylic acids, such as oxalic acid, malonic acid, succinic acid, maleic acid, fumaric acid, adipic acid, butanetetracarboxylic acid, isophthalic acid, terephthalic acid, trimellitic acid, and pyromellitic acid.

Examples of the carbamic acids having a total of from 2 to 20 carbon atoms including a carbon atom(s) of a carbonyl group(s) as described herein include *N,N-*diethylcarbamic acid, *N*-carboxypyrrolidone, *N*-carboxyaniline, and *N*,*N*-dicarboxy-2,4-toluenediamine.

Examples of the sulfonic acids having from 1 to 20 carbon atoms as described herein include fluorosulfonic acid, methanesulfonic acid, ethanesulfonic acid, *p*-toluenesulfonic acid, 4-ethylbenzenesulfonic acid, picrylsulfonic acid, 4-nitrobenzenesulfonic acid, 3-(*N-*morpholino)propanesulfonic acid, 2-morpholinoethanesulfonic acid, 2-naphthalenesulfonic acid, 4,4'-biphenyldisulfonic acid, 4-nitrotoluene-2-sulfonic acid, and 3-pyridinesulfonic acid.

Examples of the alcohols having from 1 to 20 carbon atoms described herein include: monohydric alcohols, such as methanol, ethanol, *n*-butyl alcohol, *sec*-butyl alcohol, *tert*-butyl alcohol, *n*-octyl alcohol, lauryl alcohol, cyclopentanol, cyclohexanol, allyl alcohol, benzyl alcohol, 1-phenylethyl alcohol, triphenyl carbinol, and cinnamyl alcohol; and polyhydric alcohols, such as ethylene glycol, propylene glycol, diethylene glycol, dipropylene glycol, 1,3-propanediol, 1,4-butanediol, 1,6-hexanediol, 1,4-cyclohexanediol, trimethylolpropane, glycerin, diglycerin, pentaerythritol, and dipentaerythritol.

Examples of the sugars and derivatives thereof described herein include glucose, sorbitol, dextrose, fructose, and sucrose.

Examples of the hydroxy group-containing aromatic compounds having from 6 to 20 carbon atoms described herein include phenol, catechol, resorcinol, hydroquinone, 1-naphthol, 2-naphthol, 1,2-dihydroxynaphthalene, 2,6-dihydroxynaphthalene, *o*-cresol, *m-*cresol, *p*-cresol, 2,4,6-trimethylphenol, 4-nitrophenol, 4-methoxyphenol, anthrarobin, 9-phenanthrol, 1-hydroxypyrene, and bisphenol A.

Examples of the polyalkylene oxides described herein include polyethylene oxide, polypropylene oxide, and copolymers thereof.

Examples of the compound having an active hydrogen atom bonded to a nitrogen atom described herein include: aliphatic primary amines having from 1 to 20 carbon atoms; aromatic primary amines having from 6 to 20 carbon atoms; aliphatic secondary amines having a total of from 2 to 20 carbon atoms; aromatic secondary amines having a total of from 6 to 20 carbon atoms; primary or secondary amino group-containing polyamines having a total of from 2 to 20 carbon atoms; saturated or unsaturated cyclic secondary amines having a total of from 4 to 20 carbon atoms; secondary amino group-containing cyclic polyamines having a total of from 4 to 20 carbon atoms; acid amides having a total of from 2 to 20 carbon atoms including a carbon atom(s) of a carbonyl group(s) obtained from a primary or secondary amine; 5- to 7-membered cyclic amides; and dicarboxylic acid imides having a total of from 4 to 10 carbon atoms including carbon atoms of carbonyl groups.

Examples of the aliphatic primary amines having from 1 to 20 carbon atoms described herein include methylamine, ethylamine, *n*-butylamine, isobutylamine, *sec-*butylamine, *tert-*butylamine, and cyclohexylamine.

Examples of the aromatic primary amines having from 6 to 20 carbon atoms as described herein include benzylamine, *β*-phenylethylamine, aniline, *o*-toluidine, *m*-toluidine, and p*-*toluidine.

Examples of the aliphatic secondary amines having a total of from 2 to 20 carbon atoms as described herein include dimethylamine, methylethylamine, diethylamine, ethyl-*n-*butylamine, methyl-*sec*-butylamine, dipentylamine, and dicyclohexylamine.

Examples of the aromatic secondary amines having a total of from 6 to 20 carbon atoms as described herein include N-methylaniline and diphenylamine.

Examples of the primary or secondary amino group-containing polyamines having a total of from 2 to 20 carbon atoms as described herein include ethylenediamine, di(2-aminoethyl)amine, hexamethylenediamine, 4,4'-diaminodiphenylmethane, tri(2-aminoethyl)amine, *N,N*-dimethylethylenediamine, *N,N*-diethylethylenediamine, and di(2-methylaminoethyl)amine.

Examples of the saturated or unsaturated cyclic secondary amines having a total of from 4 to 20 carbon atoms as described herein include pyrrolidine, piperidine, morpholine, 1,2,3,4-tetrahydroquinoline, 3-pyrroline, pyrrole, indole, carbazole, imidazole, pyrazole, and purine.

Examples of the secondary amino group-containing cyclic polyamines having a total of from 4 to 20 carbon atoms as described herein include piperazine, pyrazine, and 1,4,7-triazacyclononane.

Examples of the acid amides having a total of from 2 to 20 carbon atoms including a carbon atom(s) of a carbonyl group(s) obtained from a primary or secondary amine as described herein include acetamide, propionamide, *N*-methylpropionamide, *N-*methylbenzamide, and *N*-ethylstearamide.

Examples of the 5- to 7-membered cyclic amides as described herein include 2-pyrrolidone and ε-caprolactam.

Examples of the dicarboxylic acid imides having a total of from 4 to 10 carbon atoms including carbon atoms of carbonyl groups as described herein include succinimide, maleinimide, and phthalimide.

Examples of the compound having an active hydrogen atom bonded to a sulfur atom as described herein include monohydric thiols having from 1 to 10 carbon atoms, polyhydric thiols having from 1 to 10 carbon atoms, and aromatic mercapto compounds having from 1 to 10 carbon atoms.

Examples of the monohydric thiols having from 1 to 10 carbon atoms as described herein include methanethiol, ethanethiol, *n*-butanethiol, *tert*-butanethiol, hexanethiol, decanethiol, cyclopentyl mercaptan, and cyclohexyl mercaptan.

Examples of the polyhydric thiols having from 1 to 10 carbon atoms as described herein include 1,2-ethanedithiol, 1,3-propanedithiol, 2,3-butanedithiol, 1,6-hexanedithiol, 1,2,3-propanetrithiol, and 2,3-di(mercaptomethyl)-1,4-butanedithiol.

Examples of the aromatic mercapto compounds having from 1 to 10 carbon atoms as described herein include thiophenol, o-thiocresol, thionaphthol, and 1,2-benzenedithiol.

In Formula (1), Z⁻ is PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻).

In Formula (2), each of Y⁻ and Z⁻ independently is PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻).

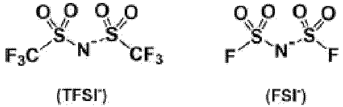

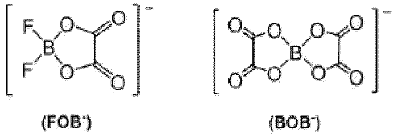

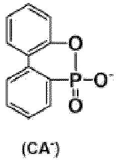

In Formula (1), each of twenty-four Rs independently represents a hydrocarbon group having from 1 to 10 carbon atoms and, among the twenty-four Rs, two Rs bonded to the same nitrogen atom are optionally bonded to each other.

In Formula (2), each of twenty-four Rs independently represents a hydrocarbon group having from 1 to 10 carbon atoms and, among the twenty-four Rs, two Rs bonded to the same nitrogen atom are optionally bonded to each other.

Examples of the hydrocarbon group having from 1 to 10 carbon atoms include aliphatic and aromatic hydrocarbon groups, such as a methyl group, an ethyl group, an n*-*butyl group, a *sec-*butyl group, a *tert*-butyl group, a 2-butenyl group, a 1-pentyl group, a 2-methyl-1-butyl group, a *tert*-pentyl group, a 3-methyl-2-butyl group, a neopentyl group, an *n*-hexyl group, a 4-methyl-2-pentyl group, a cyclopentyl group, a cyclohexyl group, a 1-octyl group, a 2-octyl group, a 2-ethyl-1-hexyl group, a 1,1-dimethyl-3,3-dimethylbutyl (commonly referred to as *tert*-octyl) group, a phenyl group, a 4-toluyl group, a benzyl group, a 1-phenylethyl group, and a 2-phenylethyl group. Thereamong, a methyl group, an ethyl group, an *n-*propyl group, an isopropyl group, a *tert*-butyl group, a *tert*-pentyl group, or a 1,1-dimethyl-3,3-dimethylbutyl group is preferred, and a methyl group is more preferred.

In Formula (1) or (2), when two Rs on the same nitrogen atom are bonded to each other to form a ring structure, the ring is preferably formed together with a divalent linear hydrocarbon group having from 4 to 6 carbon atoms in the main chain and a nitrogen atom to which this hydrocarbon group is bonded (the ring is a nitrogen atom-containing 5- to 7-membered ring), and the hydrocarbon group is preferably a tetramethylene group, a pentamethylene group, a hexamethylene group, or any of these groups in which the main chain is substituted with an alkyl group such as a methyl group or an ethyl group. Thereamong, a tetramethylene group or a pentamethylene group is more preferred.

Regarding the groups in which two Rs are bonded to a nitrogen atom in Formula (1) or (2), two Rs bonded to the same nitrogen atom may be bonded to each other to form a ring in some or all of such groups. When a ring is formed in some of the groups in which two Rs are bonded to the same nitrogen atom, two Rs in other groups in which the two Rs are bonded to the same nitrogen atom and a ring is not formed are, for example, those groups exemplified above for a case where the Rs independently represent hydrocarbon groups, and preferred groups are also the same.

In Formula (1), it is preferred that each of the twenty-four Rs independently is an alkyl group having from 1 to 3 carbon atoms and, among the twenty-four Rs, two Rs bonded to the same nitrogen atom are optionally bonded to each other.

In Formula (2), it is preferred that each of the twenty-four Rs independently is an alkyl group having from 1 to 3 carbon atoms and, among the twenty-four Rs, two Rs bonded to the same nitrogen atom are optionally bonded to each other.

In Formula (1), it is more preferred that each of the twenty four Rs is a methyl group or an ethyl group and, among the twenty-four Rs, two Rs bonded to the same nitrogen atom are optionally bonded to each other.

In Formula (2), it is more preferred that each of the twenty four Rs is a methyl group or an ethyl group and, among the twenty-four Rs, two Rs bonded to the same nitrogen atom are optionally bonded to each other.

Examples of a mode in which two of the twenty-four Rs are bonded to the same nitrogen include a mode in which the two Rs together form a tetramethylene group, a pentamethylene group, or the like.

Compounds belonging to the compound represented by Formula (1) or (2) may be used singly, or in combination of two or more kinds thereof.

The compound represented by Formula (1) or (2) can be synthesized by the method disclosed in JP-A No. H10-77289 or a similar method.

Examples of the use of the phosphazene compound (A) contained in the non-aqueous electrolyte solution for a lithium secondary battery according to the disclosure include additives, such as battery additives (preferably additives for a lithium secondary battery, more preferably additives for a non-aqueous electrolyte solution of a lithium secondary battery), reaction reagents, synthesis reaction catalysts, electrolytes for various electrochemical devices, doping agents, and lubricants.

The phosphazene compound (A) contained in the non-aqueous electrolyte solution for a lithium secondary battery according to the disclosure is particularly suitable as an additive for a non-aqueous electrolyte solution of a lithium secondary battery.

### [Non-Aqueous Electrolyte Solution for a Lithium Secondary Battery]

The non-aqueous electrolyte solution for a lithium secondary battery according to one embodiment of the disclosure (hereinafter, also simply referred to as "the non-aqueous electrolyte solution of the present embodiment") contains:
an electrolyte that is a lithium salt;
a non-aqueous solvent; and
the above-described phosphazene compound (A).

In other words, the non-aqueous electrolyte solution of the present embodiment contains:
an electrolyte that is a lithium salt;
a non-aqueous solvent; and
at least one of a first ion group and a second ion group.

The first ion group consists of cations represented by the following Formula (PZN⁺) and anions represented by (Z).

The second ion group consists of cations represented by the following Formula (PZN²⁺), anions represented by (Y⁻), and anions represented by (Z⁻).

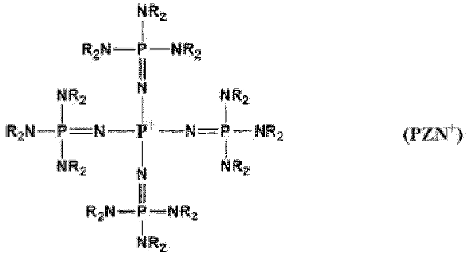

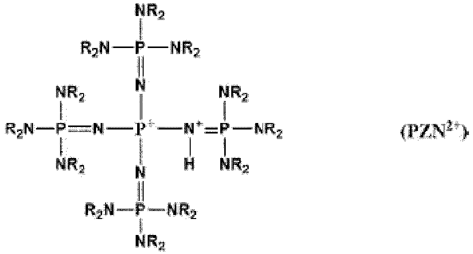

According to the non-aqueous electrolyte solution of the present embodiment, an increase in the rate of temperature increase inside a battery in the event of an internal short circuit can be inhibited.

The non-aqueous electrolyte solution of the present embodiment (hereinafter, also simply referred to as "non-aqueous electrolyte solution") contains the phosphazene compound (A).

The non-aqueous electrolyte solution of the present embodiment, by containing the phosphazene compound (A), can inhibit an increase in the rate of temperature increase inside a battery in the event of an internal short circuit.

The reason why this effect is exerted is presumed to be as follows. It is noted here, however, that the non-aqueous electrolyte solution of the present embodiment is not limited by the below-described reason.

In a lithium secondary battery containing the non-aqueous electrolyte solution of the present embodiment, when a foreign metal brought in from the outside of the battery or a metal of a metal precipitate or the like inside the battery comes into contact with both the positive electrode and the negative electrode inside the battery and causes an internal short circuit, cationic molecules of the phosphazene compound (A) contained in the non-aqueous electrolyte solution adsorb to the surface of the metal to form a coating film on the metal. The formation of this coating film increases the electronic resistance of the contact surfaces between the foreign metal or metal precipitate and the positive and negative electrodes, and this inhibits a short-circuit discharge accompanied by heat generation in the early stage of the short circuit. It is believed that, as a result, an increase in the rate of temperature increase inside the battery during the internal short circuit is inhibited.

For the above-described reason, it is believed that an increase in the rate of temperature increase inside a battery in the event of an internal short circuit can be inhibited by the non-aqueous electrolyte solution of the present embodiment.

The non-aqueous electrolyte solution of the present embodiment may contain only one of either of a phosphazene compound represented by Formula (1) or a phosphazene compound represented by Formula (2), may contain two or more of either of these compounds, or may contain both of these compounds for a total of at least two or more thereof.

A content of the phosphazene compound (A) in the non-aqueous electrolyte solution of the present embodiment is not particularly limited; however, it is preferably from 0.1% by mass to 5.0% by mass with respect to a total amount of the non-aqueous electrolyte solution.

When the content of the phosphazene compound (A) is 0.1% by mass or more with respect to a total amount of the non-aqueous electrolyte solution, the effect of the non-aqueous electrolyte solution of the present embodiment is exerted more effectively. The content of the phosphazene compound (A) is more preferably not less than 0.2% by mass, still more preferably not less than 0.3% by mass, yet still more preferably not less than 0.5% by mass, further more preferably not less than 1.5% by mass, with respect to a total amount of the non-aqueous electrolyte solution.

When the content of the phosphazene compound (A) is 5.0% by mass or less with respect to a total amount of the non-aqueous electrolyte solution, the chemical stability of the non-aqueous electrolyte solution is further improved.

The content of the phosphazene compound (A) is more preferably 3.0% by mass or less, still more preferably 2.5% by mass or less, yet still more preferably 2.0% by mass or less, with respect to a total amount of the non-aqueous electrolyte solution.

When a non-aqueous electrolyte solution recovered by disassembling a battery is actually analyzed, the amount of the phosphazene compound (A) may be less than the amount added to the non-aqueous electrolyte solution. Therefore, when even a small amount of the phosphazene compound (A) is detectable in a non-aqueous electrolyte solution extracted from a battery, the non-aqueous electrolyte solution is included in the scope of the non-aqueous electrolyte solution of the present embodiment.

Further, even if the phosphazene compound (A) is not detectable from a non-aqueous electrolyte solution, when a compound derived from a decomposition product of the phosphazene compound (A) is detected in the non-aqueous electrolyte solution or a coating film of an electrode, the non-aqueous electrolyte solution is deemed to be included in the scope of the non-aqueous electrolyte solution of the present embodiment.

These measures are also the same for compounds other than the phosphazene compound (A) that may be contained in the non-aqueous electrolyte solution.

### <Electrolyte>

The non-aqueous electrolyte solution of the present embodiment contains at least one electrolyte that is a lithium salt.

Examples of the electrolyte include:
inorganic acid anion salts, such as lithium hexafluorophosphate (LiPF₆), lithium tetrafluoroborate (LiBF₄), lithium hexafluoroarsenate (LiAsF₆), lithium hexafluorotantalate (LiTaF₆), lithium perchlorate (LiClO₄), lithium tetrachloroaluminate (LiAlCl₄), and lithium decachlorodecaborate (Li₂B₁₀Cl₁₀); and
organic acid anion salts, such as lithium trifluoromethane sulfonate (LiCF₃SO₃), lithium bis(trifluoromethanesulfonyl)imide (Li(CF₃SO₂)₂N), lithium bis(fluorosulfonyl)imide (Li(FSO₂)₂N), and lithium bis(pentafluoroethanesulfonyl)imide (Li(C₂F₅SO₂)₂N).

The lithium salt is particularly preferably LiPF₆.

The ratio of the lithium salt with respect to all electrolytes contained in the non-aqueous electrolyte solution of the present embodiment is preferably from 50% by mass to 100% by mass, more preferably from 60% by mass to 100% by mass, still more preferably from 80% by mass to 100% by mass.

The ratio of LiPF₆ with respect to all electrolytes contained in the non-aqueous electrolyte solution of the present embodiment is preferably from 50% by mass to 100% by mass, more preferably from 60% by mass to 100% by mass, still more preferably from 80% by mass to 100% by mass.

The concentration of the electrolyte in the non-aqueous electrolyte solution of the present embodiment is preferably from 0.1 mol/L to 3.0 mol/L, more preferably from 0.5 mol/L to 2.0 mol/L.

The concentration of LiPF₆ in the non-aqueous electrolyte solution of the present embodiment is preferably from 0.1 mol/L to 3.0 mol/L, more preferably from 0.5 mol/L to 2.0 mol/L.

### <Non-Aqueous Solvent>

The non-aqueous electrolyte solution of the present embodiment contains at least one non-aqueous solvent.

Examples of the non-aqueous solvent include cyclic carbonates, fluorine-containing cyclic carbonates, chain carbonates, fluorine-containing chain carbonates, aliphatic carboxylic acid esters, fluorine-containing aliphatic carboxylic acid esters, *γ*-lactones, fluorine-containing *γ*-lactones, cyclic ethers, fluorine-containing cyclic ethers, chain ethers, fluorine-containing chain ethers, nitriles, amides, lactams, nitromethane, nitroethane, sulfolane, trimethyl phosphate, dimethyl sulfoxide, and dimethyl sulfoxide phosphate.

Examples of the cyclic carbonates include ethylene carbonate (EC), propylene carbonate (PC), and butylene carbonate (BC).

Examples of the fluorine-containing cyclic carbonates include fluoroethylene carbonate (FEC).

Examples of the chain carbonates include dimethyl carbonate (DMC), diethyl carbonate (DEC), ethyl methyl carbonate (EMC), methyl propyl carbonate (MPC), ethyl propyl carbonate (EPC), and dipropyl carbonate (DPC).

Examples of the aliphatic carboxylic acid esters include methyl formate, methyl acetate, methyl propionate, methyl butyrate, methyl isobutyrate, methyl trimethylbutyrate, ethyl formate, ethyl acetate, ethyl propionate, ethyl butyrate, ethyl isobutyrate, and ethyl trimethylbutyrate.

Examples of the *γ*-lactones include *γ*-butyrolactone and *γ*-valerolactone.

Examples of the cyclic ethers include tetrahydrofuran, 2-methyltetrahydrofuran, tetrahydropyran, 1,3-dioxolan, 4-methyl-1,3-dioxolan, 1,3-dioxane, and 1,4-dioxane.

Examples of the chain ethers include 1,2-ethoxyethane (DEE), ethoxymethoxyethane (EME), diethyl ether, 1,2-dimethoxyethane, and 1,2-dibutoxyethane.

Examples of the nitriles include acetonitrile, glutaronitrile, adiponitrile, methoxyacetonitrile, and 3-methoxypropionitrile.

Examples of the amides include *N*,*N-*dimethylformamide.

Examples of the lactams include N-methylpyrrolidinone, N-methyloxazolidinone, and *N*,*N*'-dimethylimidazolidinone.

The non-aqueous solvent preferably contains at least one selected from the group consisting of cyclic carbonates, fluorine-containing cyclic carbonates, chain carbonates, and fluorine-containing chain carbonates.

In this case, a total ratio of the cyclic carbonates, the fluorine-containing cyclic carbonates, the chain carbonates, and the fluorine-containing chain carbonates in the non-aqueous solvent is preferably from 50% by mass to 100% by mass, more preferably from 60% by mass to 100% by mass, still more preferably from 80% by mass to 100% by mass.

Further, the non-aqueous solvent preferably contains at least one selected from the group consisting of cyclic carbonates and chain carbonates.

In this case, a total ratio of the cyclic carbonates and the chain carbonates in the non-aqueous solvent is preferably from 50% by mass to 100% by mass, more preferably from 60% by mass to 100% by mass, still more preferably from 80% by mass to 100% by mass.

The ratio of the non-aqueous solvent in the non-aqueous electrolyte solution of the present embodiment is preferably not less than 60% by mass, more preferably not less than 70% by mass.

An upper limit of the ratio of the non-aqueous solvent in the non-aqueous electrolyte solution of the present embodiment varies depending on a content of other components (e.g., phosphazene compound (A) and electrolyte); however, the upper limit is, for example, 99% by mass, preferably 97% by mass, still more preferably 90% by mass.

From the standpoint of further improving the dissociation of the electrolyte and the ion mobility, the non-aqueous solvent preferably has an intrinsic viscosity of 10.0 mPa·s or less at 25°C.

### <Cyclic Carbonic Acid Ester Having Unsaturated Bond>

From the standpoint of further improving the chemical stability of the non-aqueous electrolyte solution of the present embodiment, at least one cyclic carbonic acid ester having an unsaturated bond may be incorporated into the non-aqueous electrolyte solution.

The cyclic carbonic acid ester having an unsaturated bond is, for example, a vinylene carbonate compound, a vinylethylene carbonate compound, or a methylene ethylene carbonate compound.

Examples of the vinylene carbonate compound include vinylene carbonate (other name: 1,3-dioxol-2-one), methylvinylene carbonate (other name: 4-methyl-1,3-dioxol-2-one), ethylvinylene carbonate (other name: 4-ethyl-1,3-dioxol-2-one), 4,5-dimethyl-1,3-dioxol-2-one, 4,5-diethyl-1,3-dioxol-2-one, 4-fluoro-1,3-dioxol-2-one, and 4-trifluoromethyl-1,3-dioxol-2-one.

Examples of the vinylethylene carbonate compound include vinylethylene carbonate (other name: 4-vinyl-1,3-dioxolan-2-one), 4-methyl-4-vinyl-1,3-dioxolan-2-one, 4-ethyl-4-vinyl-1,3-dioxolan-2-one, 4-n-propyl-4-vinyl-1,3-dioxolan-2-one, 5-methyl-4-vinyl-1,3-dioxolan-2-one, 4,4-divinyl-1,3-dioxolan-2-one, and 4,5-divinyl-1,3-dioxolan-2-one.

Examples of the methylene ethylene carbonate compound include 4-methylene-1,3-dioxolan-2-one, 4,4-dimethyl-5-methylene-1,3-dioxolan-2-one, and 4,4-diethyl-5-methylene-1,3-dioxolan-2-one.

The cyclic carbonic acid ester having an unsaturated bond is particularly preferably vinylene carbonate.

When the non-aqueous electrolyte solution of the present embodiment contains a cyclic carbonic acid ester having an unsaturated bond, a content thereof is preferably from 0.1% by mass to 10.0% by mass, more preferably from 0.2% by mass to 5.0% by mass, still more preferably from 0.3% by mass to 3.0% by mass, with respect to a total amount of the non-aqueous electrolyte solution.

When the non-aqueous electrolyte solution of the present embodiment contains vinylene carbonate, a content thereof is preferably from 0.1% by mass to 5.0% by mass, more preferably from 0.2% by mass to 4.0% by mass, still more preferably from 0.3% by mass to 3.0% by mass, yet still more preferably from 0.3% by mass to 2.0% by mass, with respect to a total amount of the non-aqueous electrolyte solution.

When the non-aqueous electrolyte solution of the present embodiment contains a cyclic carbonic acid ester having an unsaturated bond, a ratio of a mass content of the cyclic carbonic acid ester having an unsaturated bond with respect to a mass content of the phosphazene compound (A) (hereinafter, also referred to as "mass content ratio [cyclic carbonic acid ester having an unsaturated bond/phosphazene compound (A)]") is preferably 0.05 or higher, more preferably 0.10 or higher, still more preferably 0.50 or higher.

When the mass content ratio [cyclic carbonic acid ester having an unsaturated bond/phosphazene compound (A)] is 0.05 or higher, an increase in the rate of temperature increase inside a battery in the event of an internal short circuit can be inhibited effectively.

An upper limit of the mass content ratio [cyclic carbonic acid ester having an unsaturated bond/phosphazene compound (A)] is not particularly limited; however, from the standpoint of more effectively obtaining the effect exerted by the cyclic carbonic acid ester having an unsaturated bond, it is preferably 30 or lower, more preferably 15 or lower.

### <Other Components>

The non-aqueous electrolyte solution of the present embodiment may also contain at least one other component in addition to the above-described components.

The other component is, for example, a sultone (i.e. a cyclic sulfonic acid ester) or an acid anhydride.

Examples of the sultone include propane sultone and propene sultone. These sultones may be used singly, or in combination of plural kinds thereof as a mixture.

The sultone is preferably propene sultone.

When the non-aqueous electrolyte solution of the present embodiment contains a sultone, a content thereof is preferably from 0.1% by mass to 3.0% by mass, more preferably from 0.1% by mass to 1.0% by mass, with respect to a total amount of the non-aqueous electrolyte solution.

Examples of the acid anhydride include:
carboxylic acid anhydrides, such as succinic anhydride, glutaric anhydride, and maleic anhydride;
disulfonic acid anhydrides, such as ethanedisulfonic anhydride and propanedisulfonic anhydride; and
anhydrides of a carboxylic acid and a sulfonic acid, such as sulfobenzoic anhydride, sulfopropionic anhydride, and sulfobutyric anhydride.

These acid anhydrides may be used singly, or in combination of plural kinds thereof as a mixture.

The acid anhydride is preferably sulfobenzoic anhydride.

When the non-aqueous electrolyte solution of the present embodiment contains an acid anhydride, a content thereof is preferably from 0.1% by mass to 3.0% by mass, more preferably from 0.5% by mass to 3.0% by mass, with respect to a total amount of the non-aqueous electrolyte solution.

From the standpoint of further improving the dissociation of the electrolyte and the ion mobility, the non-aqueous electrolyte solution of the present embodiment preferably has an intrinsic viscosity of 10.0 mPa·s or less at 25°C.

### <Method of Producing Non-Aqueous Electrolyte Solution>

A method of producing the non-aqueous electrolyte solution of the present embodiment is not particularly limited. The non-aqueous electrolyte solution of the present embodiment may be produced by mixing the respective components.

One example of the method of producing the non-aqueous electrolyte solution of the present embodiment is a production method that includes:
the step of dissolving an electrolyte in a non-aqueous solvent to obtain a solution;
   and
the step of adding a phosphazene compound (A) (and other additives if necessary) to the thus obtained solution and mixing the resultant to obtain a non-aqueous electrolyte solution.

### [Lithium Secondary Battery Precursor]

The lithium secondary battery precursor according to one embodiment of the disclosure (hereinafter, also referred to as "the battery precursor of the present embodiment") is a lithium secondary battery precursor that includes:
a casing; and
a positive electrode, a negative electrode, a separator, and an electrolyte solution, which are housed in the casing,
wherein
the positive electrode is configured to occlude and to release lithium ions,
the negative electrode is configured to occlude and to release lithium ions, and
the electrolyte solution comprises the above-described non-aqueous electrolyte solution of the present embodiment.

The term "lithium secondary battery precursor" used herein means a lithium secondary battery that has not been charged or discharged.

The below-described lithium secondary battery of the present embodiment is manufactured by housing a positive electrode, a negative electrode, a separator, and a non-aqueous electrolyte solution in a casing to produce a lithium secondary battery precursor, and subsequently subjecting the thus obtained lithium secondary battery precursor to charging and discharging (preferably an activation treatment that includes charging and discharging).

The battery precursor of the present embodiment includes the non-aqueous electrolyte solution of the present embodiment.

Therefore, the battery precursor of the present embodiment exerts the same effect as the non-aqueous electrolyte solution of the present embodiment.

### <Casing>

The casing in the battery precursor of the present embodiment is not particularly limited and may be, for example, a casing of a known lithium secondary battery.

Examples of such a casing include a casing that includes a laminated film, and a casing composed of a battery can and a battery can lid.

### <Positive Electrode>

The positive electrode in the battery precursor of the present embodiment is a positive electrode capable of occluding and releasing lithium ions.

The positive electrode in the battery precursor of the present embodiment preferably contains at least one positive electrode active material capable of occluding and releasing lithium ions.

The positive electrode in the battery precursor of the present embodiment more preferably includes: a positive electrode current collector; and a positive electrode mixture layer that contains a positive electrode active material and a binder.

The positive electrode mixture layer is arranged on at least a portion of the surface of the positive electrode current collector.

### (Positive Electrode Active Material)

The positive electrode active material is not particularly limited as long as it is a substance capable of occluding and releasing lithium ions, and may be any positive electrode active material that is usually used in a lithium secondary battery.

Examples of the positive electrode active material include:
oxides containing lithium (Li) and nickel (Ni) as constituent metal elements; and
oxides containing Li, Ni, and at least one metal element other than Li and Ni (e.g., a transition metal element or a main-group metal element) as constituent metal elements.

In these oxides, a metal element other than Li and Ni is preferably contained at a ratio equivalent to or lower than that of Ni in terms of the number of atoms.

The metal element other than Li and Ni may be, for example, at least one selected from the group consisting of Co, Mn, Al, Cr, Fe, V, Mg, Ca, Na, Ti, Zr, Nb, Mo, W, Cu, Zn, Ga, In, Sn, La, and Ce. These positive electrode active materials may be used singly, or in combination of two or more kinds thereof.

The positive electrode active material preferably contains a lithium-containing composite oxide represented by the following general formula (C1) (hereinafter, also referred to as "NCM").

The lithium-containing composite oxide (C1) is advantageous in that it has a high energy density per unit volume and excellent thermal stability.

LiNiₐCo_{b}Mn_{c}O₂ (C1)

[wherein, each of a, b, and c independently represents a number larger than 0 but smaller than 1, and a sum of a, b, and c is from 0.99 to 1.00].

Specific examples of the NCM include LiNi_{0.33}Co_{0.33}Mn_{0.33}O₂, LiNi_{0.5}Co_{0.2}Mn_{0.3}O₂, LiNi_{0.6}Co_{0.2}Mn_{0.2}O₂, and LiNi_{0.8}Co_{0.1}Mn_{0.1}O₂.

The positive electrode active material may also contain a lithium-containing composite oxide represented by the following general formula (C2) (hereinafter, also referred to as "NCA").

LiₜNi_{1-x-y}CoₓAl_{y}O₂ (C2)

(wherein, t represents a number of from 0.95 to 1.15, x represents a number of from 0 to 0.3, y represents a number of from 0 to 0.2, and a sum of x and y is less than 0.5)

Specific example of the NCA include LiNi_{0.80}Co_{0.15}Al_{0.05}O₂.

When the positive electrode in the battery precursor of the present embodiment includes a positive electrode current collector and a positive electrode mixture layer that contains a positive electrode active material and a binder, a content of the positive electrode active material in the positive electrode mixture layer is, for example, not less than 10% by mass, preferably not less than 30% by mass, still more preferably not less than 50% by mass, particularly preferably not less than 70% by mass, with respect to a total amount of the positive electrode mixture layer.

Further, the content of the positive electrode active material in the positive electrode mixture layer is, for example, 99.9% by mass or less, preferably 99% by mass or less.

### (Binder)

Examples of the binder that may be contained in the positive electrode mixture layer include polyvinyl acetate, polymethyl methacrylate, nitrocellulose, fluorine resins, and rubber particles.

Examples of the fluorine resins include polytetrafluoroethylenes (PTFE), polyvinylidene fluorides (PVDF), tetrafluoroethylene-hexafluoropropylene copolymers (FEP), and vinylidene fluoride-hexafluoropropylene copolymers.

Examples of the rubber particles include styrene-butadiene rubber particles and acrylonitrile rubber particles.

Thereamong, from the standpoint of improving the oxidation resistance of the positive electrode mixture layer, the binder is preferably a fluorine resin.

The above binders may be used singly, or in combination of two or more kinds thereof if necessary.

From the standpoint of satisfying both the physical properties of the positive electrode mixture layer (e.g., electrolyte solution permeability and peeling strength) and the battery performance, a content of the binder in the positive electrode mixture layer is preferably from 0.1% by mass to 4.0% by mass with respect to a total amount of the positive electrode mixture layer.

When the content of the binder is 0.1% by mass or more, the adhesion of the positive electrode mixture layer to the positive electrode current collector and the bindability between positive electrode active materials are further improved.

When the content of the binder is 4.0% by mass or less, the amount of the positive electrode active material in the positive electrode mixture layer can be further increased, so that the battery capacity is further improved.

### (Conductive Aid)

When the positive electrode in the battery precursor of the present embodiment includes a positive electrode current collector and a positive electrode mixture layer, the positive electrode mixture layer preferably contains a conductive aid.

As the conductive aid, any known conductive aid can be used.

As the conductive aid, any known conductive aid can be used.

The known conductive aid is not particularly limited as long as it is a conductive carbon material and, for example, graphites, carbon blacks, conductive carbon fibers (carbon nanotubes, carbon nanofibers, or carbon fibers), and fullerenes may be used singly, or in combination of two or more kinds thereof.

Examples of commercially available carbon black include: TOKA BLACK #4300, #4400, #4500, #5500 and the like (furnace blacks manufactured by Tokai Carbon Co., Ltd.); PRINTEX L and the like (furnace blacks manufactured by Degussa-Hüls AG); RAVEN 7000, 5750, 5250, 5000 ULTRA III, 5000 ULTRA and the like, CONDUCTEX SC ULTRA, CONDUCTEX 975ULTRA and the like, PURE BLACK 100, 115, 205 and the like (furnace blacks manufactured by Columbian Chemicals Company, Inc.); #2350, #2400B, #2600B, #30050B, #3030B, #3230B, #3350B, #3400B, #5400B and the like (furnace blacks manufactured by Mitsubishi Chemical Corporation); MONARCH 1400, 1300, 900, VULCAN XC-72R, BLACK PEARLS 2000, LITX-50, LITX-200 and the like (furnace blacks manufactured by Cabot Corporation); ENSACO 250G, ENSACO 260G, ENSACO 350G, and SUPER-P (manufactured by Timcal Ltd.); KETJEN BLACK EC-300J and EC-600JD (manufactured by AkzoNobel N.V.); and DENKA BLACK, DENKA BLACK HS-100, and FX-35 (acetylene blacks manufactured by Denka Co., Ltd.).

Examples of the graphites include, but not limited to: artificial graphites and natural graphites (e.g., flake graphite, bulk graphite, and earthy graphite).

### (Other Components)

When the positive electrode in the battery precursor of the present embodiment includes a positive electrode current collector and a positive electrode mixture layer, the positive electrode mixture layer may also contain other components in addition to the above-described components.

Examples of the other components include a thickening agent, a surfactant, a dispersant, a wetting agent, and an antifoaming agent.

### (Positive Electrode Current Collector)

As the positive electrode current collector, various materials can be used and, for example, a metal or an alloy is used.

More specific examples of the material of the positive electrode current collector include aluminum, nickel, and SUS. Thereamong, from the standpoint of the balance between the degree of conductivity and the cost, the positive electrode current collector is preferably made of aluminum. The term "aluminum" used herein means pure aluminum or an aluminum alloy.

The positive electrode current collector is particularly preferably an aluminum foil.

The aluminum foil is not particularly limited, and examples thereof include A1085 and A3003.

### (Method of Forming Positive Electrode Mixture Layer)

The positive electrode mixture layer can be formed by, for example, applying and drying a positive electrode mixture slurry containing the positive electrode active material and the binder onto the surface of the positive electrode current collector.

As a solvent contained in the positive electrode mixture slurry, an organic solvent such as N-methyl-2-pyrrolidone (NMP) is preferred.

An application method and a drying method that are employed for applying and drying the positive electrode mixture slurry onto the positive electrode current collector are not particularly limited.

Examples of the application method include slot die coating, slide coating, curtain coating, and gravure coating.

Examples of the drying method include: drying with warm air, hot air, or lowhumidity air; vacuum-drying; drying by irradiation with infrared radiation (e.g., far-infrared radiation).

The drying time and the drying temperature are not particularly limited; however, the drying time is, for example, from 1 minute to 30 minutes, and the drying temperature is, for example, from 40°C to 80°C.

A method of producing the positive electrode mixture layer preferably includes the step of, after applying and drying the positive electrode mixture slurry onto the surface of the positive electrode current collector, reducing the porosity of the resulting positive electrode active material layer by a press treatment using a mold press, a roll press, or the like.

### <Negative Electrode>

The negative electrode in the battery precursor of the present embodiment is a negative electrode capable of occluding and releasing lithium ions.

The negative electrode in the battery precursor of the present embodiment preferably contains at least one negative electrode active material capable of occluding and releasing lithium ions.

The negative electrode in the battery precursor of the present embodiment more preferably includes: a negative electrode current collector; and a negative electrode mixture layer that contains a negative electrode active material and a binder.

The negative electrode mixture layer is arranged on at least a portion of the surface of the negative electrode current collector.

### (Negative Electrode Active Material)

The negative electrode active material is not particularly limited as long as it is a substance capable of occluding and releasing lithium ions and, for example, at least one selected from the group consisting of metal lithium, lithium-containing alloys, metals and alloys that can be alloyed with lithium, oxides capable of doping and dedoping lithium ions, transition metal nitrides capable of doping and dedoping lithium ions, and carbon materials capable of doping and dedoping lithium ions (these materials may be used singly, or in combination of two or more kinds thereof as a mixture) can be used.

Thereamong, the negative electrode active material is preferably a carbon material capable of doping and dedoping lithium ions.

Examples of the carbon material include carbon black, activated charcoal, graphite materials (e.g., artificial graphites and natural graphites), and amorphous carbon materials.

The form of the carbon material may be any of a fibrous form, a spherical form, a potato form, and a flake form.

The particle size of the carbon material is not particularly limited; however, it is, for example, from 5 µm to 50 µm, preferably from 10 µm to 30 µm.

Specific examples of the amorphous carbon materials include hard carbon, cokes, mesocarbon microbeads (MCMB) calcined at 1,500°C or lower, and mesophase pitch carbon fibers (MCF).

Examples of the graphite materials include natural graphites and artificial graphites.

As the artificial graphites, for example, graphitized MCMB and graphitized MCF can be used.

As the graphite materials, for example, boron-containing graphite materials can also be used.

Further, as the graphite materials, graphite materials coated with a metal such as gold, platinum, silver, copper, or tin, graphite materials coated with amorphous carbon, and mixtures of amorphous carbon and graphite can be used as well.

These carbon materials may be used singly, or in combination of two or more kinds thereof as a mixture.

### (Binder)

As the binder, one or a combination of two or more selected from styrene-butadiene rubbers (SBR), acrylonitrile-butadiene rubbers, acrylonitrile-butadiene-styrene rubbers, carboxymethylcellulose (CMC), hydroxypropylmethylcellulose, polyvinyl alcohols, hydroxypropylcellulose, and diacetylcellulose can be used.

As the binder for the negative electrode mixture layer, it is desired to use a combination of a styrene-butadiene rubber and carboxylmethylcellulose that are mixed as appropriate.

From the standpoint of satisfying both the physical properties of the negative electrode mixture layer (e.g., electrolyte solution permeability and peeling strength) and the battery performance, the content of the binder in the negative electrode mixture layer is preferably from 0.1% by mass to 4.0% by mass with respect to a total amount of the negative electrode mixture layer.

When the content of the binder is 0.1% by mass or more, the adhesion of the negative electrode mixture layer to the negative electrode current collector and the bindability between negative electrode active materials are further improved.

When the content of the binder is 4.0% by mass or less, the amount of the negative electrode active material in the negative electrode mixture layer can be further increased, so that the battery capacity is further improved.

### (Conductive Aid)

When the negative electrode in the battery precursor of the present embodiment includes a negative electrode current collector and a negative electrode mixture layer, the negative electrode mixture layer preferably contains a conductive aid.

As the conductive aid, any known conductive aid can be used.

Specific examples of the conductive aid that may be contained in the negative electrode mixture layer are the same as the above-described specific examples of the conductive aid that may be contained in the positive electrode mixture layer.

### (Other Components)

When the negative electrode in the battery precursor of the present embodiment includes a negative electrode current collector and a negative electrode mixture layer, the negative electrode mixture layer may also contain other components in addition to the above-described components.

Examples of the other components include a thickening agent, a surfactant, a dispersant, a wetting agent, and an antifoaming agent.

### (Method of Forming Negative Electrode Mixture Layer)

The negative electrode mixture layer can be formed by, for example, applying and drying a negative electrode mixture slurry containing the negative electrode active material and the binder onto the surface of the negative electrode current collector.

As a solvent contained in the negative electrode mixture slurry, water is preferably used and, if necessary, for example, a liquid medium compatible with water may also be used for improving the applicability to the current collector.

Examples of the liquid medium compatible with water include alcohols, glycols, cellosolves, aminoalcohols, amines, ketones, carboxylic acid amides, phosphoric acid amides, sulfoxides, carboxylic acid esters, phosphoric acid esters, ethers, and nitriles, and these liquid media may be used in a range where they are compatible with water.

A preferred mode of the method of forming the negative electrode mixture layer is the same as the above-described preferred mode of the method of forming the positive electrode mixture layer.

### <Separator>

Examples of the separator in the battery precursor of the present embodiment include porous plates containing a resin such as a polyethylene (PE), a polypropylene (PP), a polyester, cellulose, or a polyamide. Examples of the separator also include nonwoven fabrics containing any of the above-described resins.

Preferred examples of the separator include porous resin sheets having a monolayer or multilayer structure mainly composed of one or more polyolefin resins.

The thickness of the separator can be set at, for example, from 5 µm to 30 µm.

The separator is preferably arranged between the positive electrode and the negative electrode.

### <Electrolyte Solution>

The electrolyte solution contained in the battery precursor of the present embodiment is the above-described non-aqueous electrolyte solution of the present embodiment.

A preferred mode of the non-aqueous electrolyte solution of the present embodiment is as described above.

### <Method of Producing Battery Precursor>

A method of producing the battery precursor of the present embodiment is not particularly limited.

One example of the method of producing the battery precursor of the present embodiment includes the step of housing the positive electrode, the negative electrode, the separator, and the electrolyte solution in the casing.

This example preferably includes:
the step of housing the positive electrode, the negative electrode, and the separator in the casing; and
the step of injecting the electrolyte solution into the casing in which the positive electrode, the negative electrode, and the separator are housed.

### <One Example of Lithium Secondary Battery Precursor>

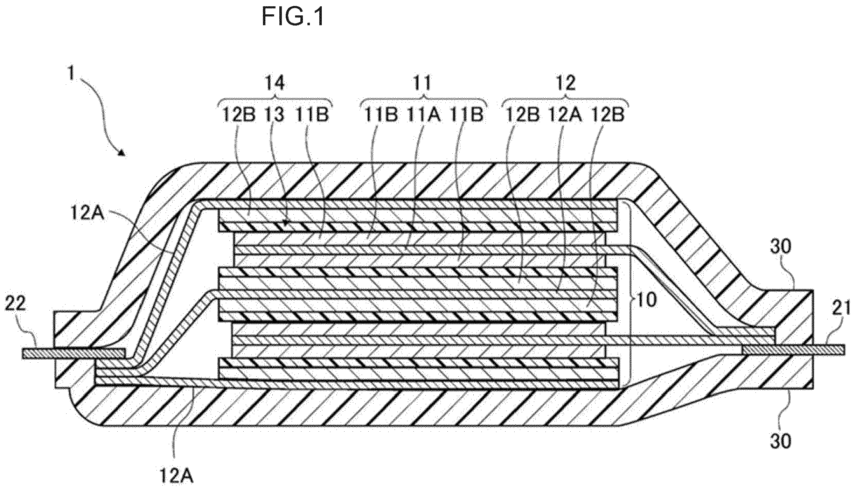

One example of the lithium secondary battery precursor of the present embodiment will now be described referring to FIG. 1; however, the lithium secondary battery precursor of the present embodiment is not limited to the below-described example.

FIG. 1 is a schematic cross-sectional view that illustrates a lithium secondary battery precursor 1, which is one example of the lithium secondary battery precursor of the present embodiment.

The lithium secondary battery precursor 1 is one example of a stacked-type lithium secondary battery.

Examples of the lithium secondary battery precursor of the present embodiment also include a wound-type lithium secondary battery in addition to the stacked-type lithium secondary battery. The wound-type lithium secondary battery has a structure in which the positive electrode, the separator, the negative electrode, and the separator are disposed on one another in this order and wound in a layered form.

As illustrated in FIG. 1, the lithium secondary battery precursor 1 has a structure in which a battery element 10, to which a positive electrode lead 21 and a negative electrode lead 22 are attached, is enclosed in an outer package 30 formed of a laminated film.

In the lithium secondary battery precursor 1, the positive electrode lead 21 and the negative electrode lead 22 are drawn out in the opposite direction from the inside of the outer package 30 to the outside.

The positive electrode lead 21 and the negative electrode lead 22 can be attached to the below-described positive electrode current collector and negative electrode current collector by, for example, ultrasonic welding or resistance welding.

It is noted here that the positive electrode lead and the negative electrode lead may be drawn out in the same direction from the inside of the outer package to the outside, although this is not illustrated.

As illustrated in FIG. 1, the battery element 10 has a structure in which a positive electrode 11 in which a positive electrode mixture layer 11B is formed on both main surfaces of a positive electrode current collector 11A, a separator 13, and a negative electrode 12 in which a negative electrode mixture layer 12B is formed on both main surfaces of a negative electrode current collector 12A are disposed in layers.

In this structure, the positive electrode mixture layer 11B formed on one of the main surfaces of the positive electrode current collector 11A of the positive electrode 11 and the negative electrode mixture layer 12B formed on one of the main surfaces of the negative electrode current collector 12A of the negative electrode 12 adjacent to the positive electrode 11 face each other via the separator 13.

Inside the outer package 30 of the lithium secondary battery precursor 1, the non-aqueous electrolyte solution of the present embodiment (not illustrated) is injected. The electrolyte solution of the present embodiment is impregnated into the positive electrode mixture layer 11B, the separator 13, and the negative electrode mixture layer 12B.

In the lithium secondary battery precursor 1, a single cell layer 14 is formed by the positive electrode mixture layer 11B, the separator 13, and the negative electrode mixture layer 12B that are adjacent to each other.

It is noted here that the positive electrode and the negative electrode may each have an active material layer formed on one side of the respective current collector.

One example of the below-described lithium secondary battery according to one embodiment of the disclosure is a lithium secondary battery in a mode that, on the surfaces of each positive electrode mixture layer 11B and negative electrode mixture layer 12B in the lithium secondary battery precursor 1, coating films are formed by charging and discharging of the lithium secondary battery precursor 1.

### [Method of Manufacturing Lithium Secondary Battery]

The method of manufacturing a lithium secondary battery according to one embodiment of the disclosure (hereinafter, also referred to as "the battery manufacturing method of the present embodiment") is a method of manufacturing a lithium secondary battery, which method includes:
the step of preparing the battery precursor of the present embodiment (hereinafter, also referred to as "the preparation step"); and
the step of performing an activation treatment of the battery precursor to obtain a lithium secondary battery (hereinafter, also referred to as "the activation step"),
wherein the activation treatment includes charging and discharging the battery precursor in an environment of from 15 to 70°C.

The activation treatment in the activation step preferably includes:
an initial retention phase in which the battery precursor is retained in an environment of from 15°C to 70°C (preferably from 25°C to 60°C);
an initial charging phase in which, after the initial retention phase, the battery precursor is charged in an environment of from 15°C to 50°C (preferably from 25°C to 45°C);
a second retention phase in which, after the initial charging phase, the battery precursor is retained in an environment of from 15°C to 70°C (preferably from 25°C to 60°C); and
a charge-discharge phase in which, after the second retention phase, the battery precursor is at least once subjected to a combination of at least one of charging or discharging in an environment of from 15°C to 50°C (preferably from 25°C to 45°C).

According to the above-described preferred aspect, the effect of inhibiting an increase in the rate of temperature increase inside a lithium secondary battery in the event of an internal short circuit is exerted more effectively.

### [Lithium Secondary Battery]

The lithium secondary battery according to one embodiment of the disclosure (hereinafter, also referred to as "the battery of the present embodiment") is a lithium secondary battery obtained by charging and discharging the above-described battery precursor (i.e. lithium secondary battery precursor), preferably a lithium secondary battery obtained by performing the above-described activation treatment of the battery precursor.

The non-aqueous electrolyte solution for a lithium secondary battery according to the disclosure, the lithium secondary battery precursor according to the disclosure, the method of manufacturing a lithium secondary battery according to the disclosure, and the lithium secondary battery according to the disclosure, which are described above, can be applied to the use in, for example, electronics such as cellular phones and laptop personal computers; electric vehicles; hybrid cars; and power sources for power storage.

The non-aqueous electrolyte solution for a lithium secondary battery according to the disclosure, the lithium secondary battery precursor according to the disclosure, the method of manufacturing a lithium secondary battery according to the disclosure, and the lithium secondary battery according to the disclosure can be particularly preferably used for hybrid cars or electric vehicles.

### [Phosphazene Compound]

The phosphazene compound according to the disclosure contains at least one of a phosphazene compound represented by the following Formula (1) or a phosphazene compound represented by the following Formula (2):

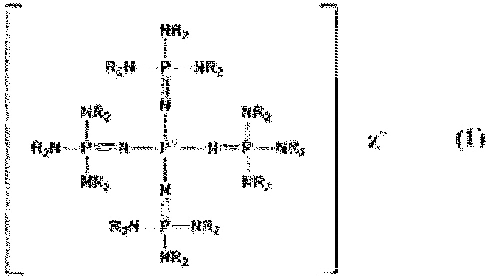

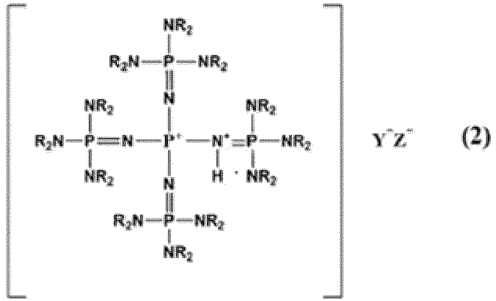

In Formula (1), Z⁻ represents PO₂F₂⁻; each of twenty-four Rs independently represents a hydrocarbon group having from 1 to 10 carbon atoms; and, among the twenty-four Rs, two Rs bonded to the same nitrogen atom are optionally bonded to each other.

In Formula (2), each of Y⁻ and Z⁻ independently represents an anion in which a proton is removed from an inorganic acid or an active hydrogen compound; each of twenty-four Rs independently represents a hydrocarbon group having from 1 to 10 carbon atoms; and, among the twenty-four Rs, two Rs bonded to the same nitrogen atom are optionally bonded to each other.

The use of the phosphazene compound according to the disclosure as a battery additive can inhibit an increase in the rate of temperature increase inside a lithium secondary battery in the event of an internal short circuit.

Examples of the phosphazene compound represented by Formula (1) in the phosphazene compound according to the disclosure include the same ones as those exemplified above for the phosphazene compound represented by Formula (1) in the phosphazene compound (A) that is contained in the non-aqueous electrolyte solution for a lithium secondary battery according to the disclosure, except that the inorganic acid does not include hydrochloric acid and the active hydrogen compound does not include water, formic acid, and acetic acid.

Examples of the phosphazene compound represented by Formula (2) in the phosphazene compound according to the disclosure include the same ones as those exemplified above for the phosphazene compound represented by Formula (2) in the phosphazene compound (A) that is contained in the non-aqueous electrolyte solution for a battery according to the disclosure.

Examples of the use of the phosphazene compound according to the disclosure include additives, such as battery additives (preferably additives for a lithium secondary battery, more preferably additives for a non-aqueous electrolyte solution of a lithium secondary battery), reaction reagents, synthesis reaction catalysts, electrolytes for various electrochemical devices, doping agents, and lubricants.

### [Lithium Secondary Battery Additive]

The lithium secondary battery additive according to the disclosure contains a phosphazene compound (A) that contains at least one of a phosphazene compound represented by the following Formula (1) or a phosphazene compound represented by the following Formula (2):

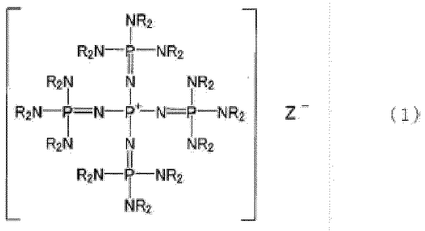

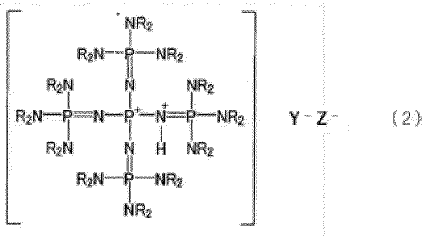

In Formula (1), Z⁻ represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other, and
in Formula (2), each of Y⁻ and Z⁻ independently represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other

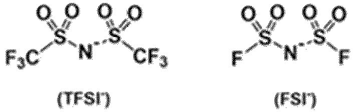

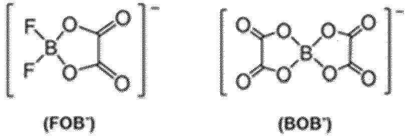

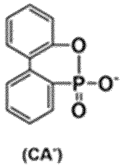

The lithium secondary battery additive according to the disclosure can inhibit an increase in the rate of temperature increase inside a lithium secondary battery in the event of an internal short circuit.

Examples of the phosphazene compound (A) contained in the lithium secondary battery additive according to the disclosure include the same ones as those exemplified above for the phosphazene compound (A) contained in the non-aqueous electrolyte solution for a lithium secondary battery according to the disclosure.

### Examples

Examples of the disclosure will now be described; however, the disclosure is not limited by the below-described Examples.

In the below-described Examples, "added amount" means the content in an eventually obtained non-aqueous electrolyte solution (i.e. amount with respect to a total amount of an eventually obtained non-aqueous electrolyte solution).

Further, "wt%" denotes "% by mass".

### [Synthesis Example 1: Synthesis of Tetrakis[tris(dimethylamino)phosphoranylideneamino]phosphonium Difluorophosphate]

Water used in the below-described Examples was water purified by "MQ ACADEMIC A10 SYSTEM" manufactured by Merck Millipore Ltd.

In a 1,000-ml flask, 4.99 g (6.44 mmol) of tetrakis[tris(dimethylamino)phosphoranylideneamino]phosphonium chloride (product number "87651" manufactured by Sigma-Aldrich Co., LLC) was weighed, and 500.0 g of water was subsequently added thereto to obtain an aqueous solution. At room temperature, the tetrakis[tris(dimethylamino)phosphoranylideneamino]phosphonium chloride was dissolved in water while stirring the aqueous solution using a magnetic stirrer, whereby a solution A was obtained.

Separately, 20.17 g of water was added to 0.765 g (7.09 mmol) of lithium difluorophosphate, and this lithium difluorophosphate was dissolved in water at room temperature, whereby a solution B was obtained.

Dropwise addition of the solution B to the solution A using a Pasteur pipette under room temperature caused turbidity. The whole amount of the solution B was added dropwise to the solution A to obtain a reaction solution. The blending ratio of lithium difluorophosphate was 1.1 times by mole with respect to tetrakis[tris(dimethylamino)phosphoranylideneamino]phosphonium chloride in terms of molar ratio.

The reaction solution was stirred at room temperature for 1 hour. Subsequently, the whole amount of the reaction solution was transferred to a pressure filtration apparatus. This pressure filtration apparatus was equipped with a membrane filter of 0.2 µm in pore size ("H020A142C" manufactured by ADVANTEC Co., Ltd., diameter: 142 mm), and had a capacity of 1 L.

The reaction solution was filtered at a nitrogen pressure of 0.2 MPa (gauge pressure) to separate white solids from the reaction solution. The thus separated white solids in the pressure filtration apparatus were washed with 100 ml of water three times. Then, the white solids were taken out of the pressure filtration apparatus and transferred to a petri dish.

The white solids were air-dried at room temperature for 2 days. Thereafter, the petri dish was placed in a desiccator, and the white solids were vacuum-dried at room temperature until a change in the mass of the white solids was no longer observed, whereby white dry solids were obtained.

The mass of the thus obtained white dry solids was 4.24 g. The yield was 78%, assuming that the whole amount of the white dry solids was tetrakis[tris(dimethylamino)phosphoranylideneamino]phosphonium difluorophosphate.

### [Analysis 1 of White Dry Solids] Analysis by ³¹P-NMR

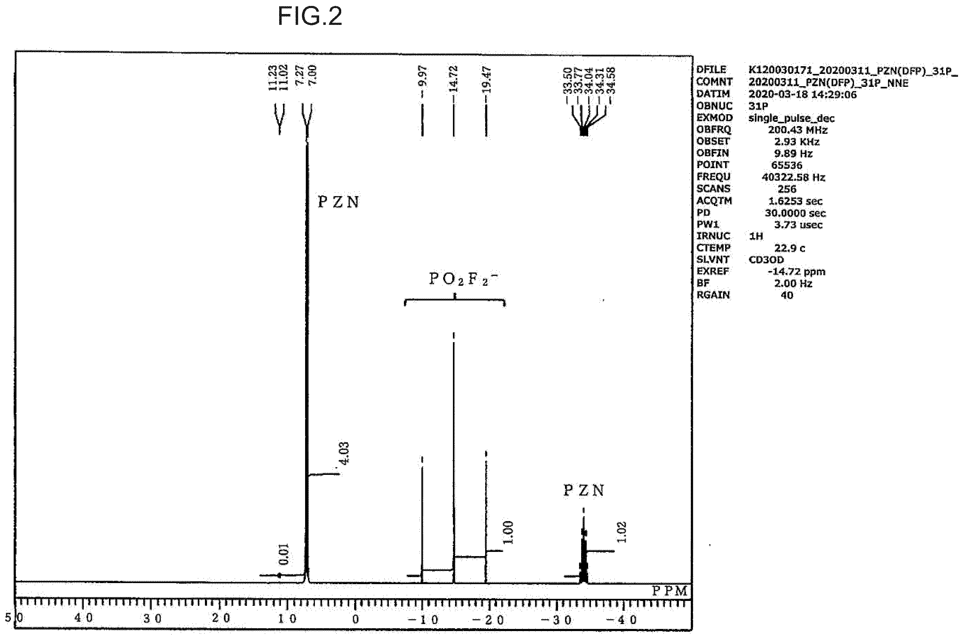

The white dry solids in an amount of 0.500 g were dissolved in 0.499 g of deuterated methanol, and ³¹P-NMR was measured using a nuclear magnetic resonance apparatus ("ECA500" manufactured by JEOL Ltd.). The thus obtained ³¹P-NMR measurement chart of the white dry solids is shown in FIG. 2.

In FIG. 2, five signals (hereinafter, referred to as "quintet") are observed at from - 33.50 ppm to -34.58 ppm. This quintet is attributed to a single phosphorus atom positioned at the center of a tetrakis[tris(dimethylamino)phosphoranylideneamino]phosphonium cation.

In addition, two signals (hereinafter, referred to as "doublet") are observed at 7.27 ppm and 7.00 ppm. This doublet is attributed to four phosphorus atoms positioned at the periphery of a tetrakis[tris(dimethylamino)phosphoranylideneamino]phosphonium cation.

Further, three signals (hereinafter, referred to as "triplet") are observed at -9.97 ppm, -14.72 ppm, and -19.47 ppm. This triplet is attributed to a single phosphorus atom of a difluorophosphate anion.

With regard to the tetrakis[tris(dimethylamino)phosphoranylideneamino]phosphonium cation, the integral value of the single central phosphorus atom attributed as described above (hereinafter, referred to as "first integral value") was 1.02. The integral value of the four peripheral phosphorus atoms attributed as described above (hereinafter, referred to as "second integral value") was 4.03. The value of [first integral value:second integral value] was substantially 1:4.

The integral value of the single phosphorus atom of the difluorophosphate anion is 1.00. Therefore, it was suggested that the tetrakis[tris(dimethylamino)phosphoranylideneamino]phosphonium cation and the difluorophosphate anion existed at a molar ratio of 1:1, forming an electrically neutral salt.

### [Analysis 2 of White Dry Solids] Measurement of Chloride Ion Content by Elemental Analysis

The above-obtained white dry solids in an amount of 0.0500 g were dissolved in methanol to prepare 10 ml of a solution. Chloride ions in this solution were analyzed using an ion chromatography system ("ICS-3000" manufactured by Thermo Fisher Scientific K.K.). As a result, the chloride ion content in the solution was found to be less than 50 µg/g.

The chloride ion content in tetrakis[tris(dimethylamino)phosphoranylideneamino]phosphonium chloride used as a raw material is 45,726 µg/g. Therefore, it was found that 99.9% by mass of the chloride ions in tetrakis[tris(dimethylamino)phosphoranylideneamino]phosphonium chloride was substituted with other anion species.

In the above-described analyses:
from the results of the ³¹P-NMR analysis, it was suggested that the tetrakis[tris(dimethylamino)phosphoranylideneamino]phosphonium cation and the difluorophosphate anion existed at a molar ratio of 1:1;
from the results of the chloride ion analysis, it was suggested that 99.9% by mass of the chloride ions in tetrakis[tris(dimethylamino)phosphoranylideneamino]phosphonium chloride used as a raw material disappeared, being substituted with other anion species; and
the white dry solids were found to be tetrakis[tris(dimethylamino)phosphoranylideneamino]phosphonium difluorophosphate.

### [Examples 1 to 11 and Comparative Examples 1 to 3]

### <Preparation of Non-Aqueous Electrolyte Solutions (Samples 0 to 13)>

### (Sample 0)

Ethylene carbonate (hereinafter, referred to as "EC") and ethyl methyl carbonate (EMC) were mixed at EC:EMC = 30:70 (volume ratio) to prepare a mixed solvent as a non-aqueous solvent.

In this mixed solvent, LiPF₆ as an electrolyte was dissolved such that the concentration thereof in a non-aqueous electrolyte solution to be eventually obtained would be 1 mol/L, and vinylene carbonate (VC) as an additive was dissolved such that the content thereof with respect to a total amount of the non-aqueous electrolyte solution to be eventually obtained would be 0.5% by mass.

In this manner, a sample 0 was obtained.

The sample 0 is a non-aqueous electrolyte solution used in the battery of Comparative Example 1.

### (Samples 1 to 11)

Samples 1 to 11 were obtained in the same manner as the sample 0, except that the compounds shown in Table 1, which are examples of the phosphazene compound (A), were further added such that the content of each compound with respect to a total amount of a non-aqueous electrolyte solution to be eventually obtained would be the respective added amounts shown in Table 1.

The samples 1 to 11 are non-aqueous electrolyte solutions used in the batteries of Examples 1 to 11, respectively.

### (Samples 12 and 13)

Samples 12 and 13 were obtained in the same manner as the sample 0, except that the comparative phosphazene compounds shown in Table 1 (hexafluorocyclotriphosphazene or hexaphenoxycyclotriphosphazene) were further added such that the content of each compound with respect to a total amount of a non-aqueous electrolyte solution to be eventually obtained would be 1.0% by mass.

The samples 12 and 13 are non-aqueous electrolyte solutions used in the batteries of Comparative Examples 2 and 3, respectively.

### <Production of Lithium Secondary Battery>

As a lithium secondary battery, a stacked-type battery (design capacity: 300 mAh) (hereinafter, also simply referred to as "battery") was produced in the following manner.

### (Production of Positive Electrode)

### 1. Slurry Preparation

A 5-L planetary disperser was used for slurry preparation.

After mixing 920 g of NCM523 (i.e. LiNi_{0.5}Co_{0.2}Mn_{0.3}O₂) as a positive electrode active material, 20 g of SUPER-P as a conductive aid (conductive carbon, manufactured by Timcal Ltd.), and 20 g of KS-6 as a conductive aid (flake graphite, manufactured by TIMREX) for 10 minutes, 100 g of *N*-methylpyrrolidone (NMP) was added, and the resultant was further mixed for 20 minutes.

Next, 150 g of a 8%-PVDF solution (obtained by dissolving PVDFW #7200 manufactured by Kureha Corporation in NMP) was added, and the resultant was kneaded for 30 minutes, followed by a further addition of 150 g of the 8%-PVDF solution and 30-minute kneading. Subsequently, 200 g of the 8%-PVDF solution was added, and the resultant was kneaded for 30 minutes. Then, 80 g of a solution dissolved in NMP was added, followed by 30-minute kneading. Thereafter, 27 g of NMP was added to adjust the viscosity, and the resultant was mixed for 30 minutes and then vacuum-degassed for 30 minutes.

In this manner, a positive electrode mixture slurry having a solid concentration of 60% was obtained.

### 2. Coating and Drying

A die coater was used for slurry coating.

The above-obtained positive electrode mixture slurry was coated and dried onto a portion of one side of an aluminum foil (thickness: 20 µm, width: 200 mm) used as a positive electrode current collector such that the coated mass after drying would be 19.0 mg/cm². Subsequently, the positive electrode mixture slurry was coated and dried onto a portion of the opposite side (uncoated surface) of the aluminum foil such that the coated mass would be 19.0 mg/cm² in the same manner.

The thus obtained aluminum foil coated on both sides (38.0 mg/cm²) was dried in a vacuum-drying oven at 130°C for 12 hours.

### 3. Pressing

A 35-ton press machine was used. The gap (space) between the upper and lower rolls was adjusted, and the above-obtained positive electrode was pressed to a press density of 2.9 ± 0.05 g/cm³.

### 4. Slitting

The electrode was slit such that an electrode coated area of 29 mm × 40 mm and a tab welding margin were obtained.

### (Production of Negative Electrode)

### 1. Slurry Preparation

A 5-L planetary disperser was used for slurry preparation.

A 1% CMC aqueous solution (i.e. a 1%-by-mass aqueous solution of carboxymethylcellulose (CMC)) in an amount of 450 g was added to 960 g of natural graphite as a negative electrode active material and 10 g of SUPER-P as a conductive aid (conductive carbon, BET specific surface area: 62 m²/g), and the resultant was mixed for 30 minutes.

After adding 300 g of the 1% CMC aqueous solution to the thus obtained mixture and kneading the resultant for 30 minutes, 250 g of the 1% CMC aqueous solution was further added, followed by 30-minute kneading.

To the thus obtained kneaded product, 50 g of a styrene-butadiene rubber (SBR) (40% emulsified liquid) was added as a binder, and the resultant was mixed for 30 minutes and then vacuum-degassed for 30 minutes.

In this manner, a negative electrode mixture slurry having a solid concentration of 45% was obtained.

### 2. Coating and Drying

A die coater was used for slurry coating.

The above-obtained negative electrode mixture slurry was coated and dried onto a portion of one side of a copper foil (thickness: 10 µm) used as a negative electrode current collector such that the coated mass after drying would be 11.0 mg/cm². Subsequently, the negative electrode mixture slurry was coated and dried onto a portion of the opposite side (uncoated surface) of the copper foil such that the coated mass would be 11.0 mg/cm².

The thus obtained copper foil coated on both sides (22.0 mg/cm²) was dried in a vacuum-drying oven at 120°C for 12 hours.

### 3. Pressing

A small press machine was used.

The gap (space) between the upper and lower rolls was adjusted, and the above-obtained negative electrode was pressed to a press density of 1.45 ± 0.05 g/cm³.

### 4. Slitting

The electrode was slit such that an electrode coated area of 31 mm × 42 mm and a tab welding margin were obtained.

### (Battery Production)

### 1. Layering

As a separator, a polyethylene porous film (50 mm × 50 mm) having a porosity of 45% and a thickness of 25 µm was used.

The negative electrode (front surface), the separator, the positive electrode (back surface/front surface), and the negative electrode (front surface) were sequentially disposed on one another and immobilized. Subsequently, using an ultrasonic bonding machine, an aluminum tab was connected to the margin of the positive electrode, and a nickel tab was connected to the margin of the negative electrode. The resultant was sandwiched between laminate sheets, and three sides were heat-sealed.

### 2. Injection of Electrolyte Solution

The thus obtained battery was dried under reduced pressure in a vacuum dryer at 70°C for 12 hours before injecting thereto an electrolyte solution. An electrolyte solution corresponding to each sample prepared above was injected in an amount of 1.20 ± 0.05 g, and the battery was subsequently heat-sealed under vacuum.

### 3. Activation Treatment

After the injection of an electrolyte solution, each battery was maintained in the atmosphere at 25°C for 24 hours, and this battery was constant-current charged at 0.1 C (0.1 C-CC) for 3 hours, followed by a 12-hour rest at 25°C.

Subsequently, the battery was subjected to constant current-constant voltage charging up to 4.2 V (SOC: 100%) at 0.1 C (0.1 C-CCCV), rested for 30 minutes, and then constant-current discharged to 2.8 V at 0.1 C (0.1 C-CC).

### <Measurement of Heat Generation Temperature of Electrolyte Solution>

For the non-aqueous electrolyte solutions of Examples and Comparative Examples (the non-aqueous electrolyte solutions obtained in the above-described preparation of non-aqueous electrolyte solutions), differential scanning calorimetry was performed using a differential scanning calorimeter ("DSC7000X" manufactured by Hitachi High-Tech Science Corporation) at a heating rate of 5°C/min in a measurement temperature range of from 40°C to 340°C.

A point at which the rate of change in heat flux, which is represented by the following Formula (a), was 50 µW/°C or higher in a range of from 200°C to 300°C was determined as the heat generation start temperature due to decomposition of each non-aqueous electrolyte solution. The heat generation start temperature is shown in Table 1.

Formula (a): Rate of change in heat flux = Amount of change in heat flux/Amount of change in temperature

### <Evaluation of Rate of Temperature Increase Inside Battery During Short Circuit>

For the batteries of Examples of Comparative Examples, a nail penetration test was conducted to evaluate the rate of temperature increase inside each battery during a short circuit.

The details are described below.

### (Nail Penetration Test)

Each stacked-type battery produced above (design capacity: 300 mAh) was subjected to constant current-constant voltage charging up to 4.2 V (SOC: 100%) at 0.1 C (0.1 C-CCCV), and a nail penetration test was conducted. A nail of 3 mm in diameter and 15 mm in length, which had a tip angle of 30° and a bottomed hole of 0.6 mm in diameter, was covered with MACOR (registered trademark). Subsequently, a sheath-type K thermocouple having a sheath diameter of 0.5 mm and a sheath length of 200 mm (product number: IP10-K-0.5-200) was inserted to the vicinity of the nail tip to obtain a nail penetration test jig.

This nail penetration test jig and the stacked-type battery were immobilized in a nail penetration tester ("TYS-94DM45" manufactured by Toyo System Co., Ltd.), and the test jig was inserted into the middle of the battery (cell) at a speed of 1 mm/sec to cause a short circuit between the positive electrode and the negative electrode inside the battery container. In this process, the temperature inside the battery and the surface temperature were measured over time by the nail penetration test jig and the thermocouple fitted to the battery surface, respectively.

### -Calculation of Heat Generation Rate-

The heat generation rate of each battery was calculated as follows from the measurement results of the above-described nail penetration test. After the short circuit, the time at which the battery internal temperature was increased by 0.2°C was defined as the heat generation start time T₀ (sec), and one second after T₀ was defined as T₁ (sec). The battery internal temperature at T₀ and the battery internal temperature at T₁ were defined as H₀ (°C) and H₁ (°C), respectively, and the heat generation rate immediately after the short circuit was calculated using the following equation.

The results thereof are shown in Table 1. It is noted here that the values shown in Table 1 are relative values [%] in the respective cases, taking the value of the heat generation rate in Comparative Example 1 as 100%.

Heat generation rate: (H₁ - H₀)/(T₁ - T₀)

### <Evaluation of Battery Performance>

For the batteries of Examples of Comparative Examples (batteries subjected to the above-described activation treatment), the initial battery resistance value was measured as follows.

### (Measurement of Initial Battery Resistance (DC-IR))

Each activation-treated battery was charged at room temperature up to a constant voltage of 4.2 V and subsequently discharged at a constant current of 0.1 C under room temperature, and a first potential drop amount in the period of 10 seconds after the start of the discharge was measured.

Similarly, each activation-treated battery was charged at room temperature up to a constant voltage of 4.2 V and subsequently discharged at a constant current of 0.2 C under room temperature, and a second potential drop amount in the period of 10 seconds after the start of the discharge was measured.

Each activation-treated battery was charged at room temperature up to a constant voltage of 4.2 V and subsequently discharged at a constant current of 0.5 C under room temperature, and a third potential drop amount in the period of 10 seconds after the start of the discharge was measured.

Each activation-treated battery was charged at room temperature up to a constant voltage of 4.2 V and subsequently discharged at a constant current of 1.0 C under room temperature, and a fourth potential drop amount in the period of 10 seconds after the start of the discharge was measured.

From the thus measured first to fourth potential drop amounts during discharge at the respective current rates, the battery resistance (direct-current resistance; DC-IR) in the initial stage of discharge was calculated.

The results thereof are shown in Table 1. It is noted here that the values shown in Table 1 are relative values (%) in the respective cases, taking the value of the initial battery resistance (DC-IR) in Comparative Example 1 as 100.

**[Table 1]**

| | Non-aqueous electrolyte solution | | | | Evaluation results | | |
|---|---|---|---|---|---|---|---|
| | Cyclic carbonic acid ester having an unsaturated bond | | Phosphazene compound (A) or comparative phosphazene compound | | Non-aqueous electrolyte solution | Lithium secondary battery | |
| | | | | | Heat generation start temperature (°C) | Relative value of heat generation rate (%) | Relative value of initial DCIR (%) |
| | Type | Added amount (wt%) | Type | Added amount (wt%) | | | |
| Comparative Example 1 | VC | 0.5 | none | - | 234 | 100 | 100 |
| Comparative Example 2 | VC | 0.5 | hexafluorocyclotriphosphazene | 1.0 | 234 | 105 | 100 |
| Comparative Example 3 | VC | 0.5 | hexaphenoxycyclotriphosphazene | 1.0 | 235 | 99 | - |
| Example 1 | VC | 0.5 | PZN-PF₆ | 0.5 | 256 | 65 | 101 |
| Example 2 | VC | 0.5 | PZN-PF₆ | 1.0 | 262 | 74 | 101 |
| Example 3 | VC | 0.5 | PZN-PF₆ | 2.0 | 269 | 60 | 103 |
| Example 4 | VC | 0.5 | PZN-PF₆ | 3.0 | 267 | 69 | 107 |
| Example 5 | VC | 0.5 | PZN-FSI | 0.5 | 256 | 66 | 100 |
| Example 6 | VC | 0.5 | PZN-TFSI | 0.5 | 252 | 48 | 101 |
| Example 7 | VC | 0.5 | PZN-BOB | 0.5 | 258 | 69 | 102 |
| Example 8 | VC | 0.5 | PZN-FOB | 0.5 | 258 | 61 | 99 |
| Example 9 | VC | 0.5 | PZN-DFP | 0.5 | 254 | 58 | 100 |
| Example 10 | VC | 0.5 | PZN-CA | 0.5 | - | 52 | - |
| Example 11 | VC | 0.5 | PZN⁺-(PF₆/TFSI) | 0.5 | - | 78 | - |

### -Description of Table 1-

- PZN-PF₆: a compound of Formula (1) in which twenty-four Rs are all methyl groups, and Z⁻ is PF₆-.
- PZN-FSI: a compound of Formula (1) in which twenty-four Rs are all methyl groups, and Z⁻ is the above-described (FSI⁻).
- PZN-TFSI: a compound of Formula (1) in which twenty-four Rs are all methyl groups, and Z⁻ is the above-described (TFSI).
- PZN-BOB: a compound of Formula (1) in which twenty-four Rs are all methyl groups, and Z⁻ is the above-described (BOB⁻).
- PZN-FOB: a compound of Formula (1) in which twenty-four Rs are all methyl groups, and Z⁻ is the above-described (FOB⁻).
- PZN-DFP: a compound of Formula (1) in which twenty-four Rs are all methyl groups, and Z⁻ is PO₂F₂⁻.
- PZN-CA: a compound of Formula (1) in which twenty-four Rs are all methyl groups, and Z⁻ is the above-described (CA⁻).
- PZN⁺-(PF₆/TFSI): a compound of Formula (2) in which twenty-four Rs are all methyl groups, Y⁻ is PF₆⁻, and Z⁻ is the above-described (TFSI⁻).

As shown in Table 1, it is seen that, in Examples where the phosphazene compound (A) of the disclosure was contained as an additive in the respective non-aqueous electrolyte solutions, the relative value (%) of the heat generation rate was smaller, i.e. an increase in the rate of temperature increase inside a battery during an internal short circuit was inhibited, as compared to Comparative Examples.

The non-aqueous electrolyte solutions of Examples 1 to 4 contained PZN-PF₆. Therefore, the non-aqueous electrolyte solutions of Examples 1 to 4 had a higher heat generation start temperature, which was in a range of from 256°C to 269°C, than the non-aqueous electrolyte solution of Comparative Example 1. As a result, it was found that the non-aqueous electrolyte solutions of Examples 1 to 4 had a higher thermal stability than the non-aqueous electrolyte solution of Comparative Example 1.

The non-aqueous electrolyte solution of Example 5 contained PZN-FSI. Therefore, the non-aqueous electrolyte solution of Example 5 had a higher heat generation start temperature, which was 256°C, than the non-aqueous electrolyte solution of Comparative Example 1. As a result, it was found that the non-aqueous electrolyte solution of Example 5 had a higher thermal stability than the non-aqueous electrolyte solution of Comparative Example 1.

The non-aqueous electrolyte solution of Example 6 contained PZN-TFSI. Therefore, the non-aqueous electrolyte solution of Example 6 had a higher heat generation start temperature, which was 252°C, than the non-aqueous electrolyte solution of Comparative Example 1. As a result, it was found that the non-aqueous electrolyte solution of Example 6 had a higher thermal stability than the non-aqueous electrolyte solution of Comparative Example 1.

The non-aqueous electrolyte solution of Example 7 contained PZN-BOB. Therefore, the non-aqueous electrolyte solution of Example 7 had a higher heat generation start temperature, which was 258°C, than the non-aqueous electrolyte solution of Comparative Example 1. As a result, it was found that the non-aqueous electrolyte solution of Example 7 had a higher thermal stability than the non-aqueous electrolyte solution of Comparative Example 1.

The non-aqueous electrolyte solution of Example 8 contained PZN-FOB. Therefore, the non-aqueous electrolyte solution of Example 8 had a higher heat generation start temperature, which was 258°C, than the non-aqueous electrolyte solution of Comparative Example 1. As a result, it was found that the non-aqueous electrolyte solution of Example 8 had a higher thermal stability than the non-aqueous electrolyte solution of Comparative Example 1.

The non-aqueous electrolyte solution of Example 9 contained PZN-DFP. Therefore, the non-aqueous electrolyte solution of Example 9 had a higher heat generation start temperature, which was 254°C, than the non-aqueous electrolyte solution of Comparative Example 1. As a result, it was found that the non-aqueous electrolyte solution of Example 9 had a higher thermal stability than the non-aqueous electrolyte solution of Comparative Example 1.

In the non-aqueous electrolyte solutions of Examples 1 to 3, the content of PZN-PF₆ was 2.0% by mass or less. Therefore, the lithium secondary batteries of Examples 1 to 3 had a relative value of initial DCIR of 103% or less. In other words, it was found that, in the lithium secondary batteries of Examples 1 to 3, the initial resistance was hardly deteriorated as opposed to the lithium secondary battery of Comparative Example 1.

## Claims

1. A non-aqueous electrolyte solution for a lithium secondary battery, the solution comprising a phosphazene compound (A) that comprises at least one of a phosphazene compound represented by the following Formula (1) or a phosphazene compound represented by the following Formula (2): 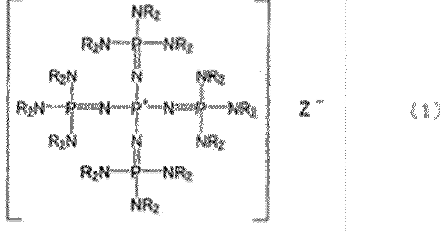 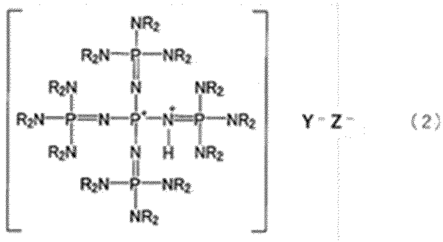
wherein, in Formula (1), Z⁻ represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other, and
in Formula (2), each of Y⁻ and Z⁻ independently represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other

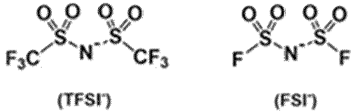

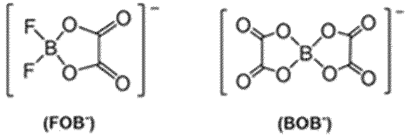

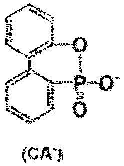

2. The non-aqueous electrolyte solution for a lithium secondary battery according to claim 1, wherein:
in Formula (1), each of the twenty-four Rs independently represents an alkyl group having from 1 to 3 carbon atoms and, among the twenty-four Rs, two Rs bonded to the same nitrogen atom are optionally bonded to each other, and
in Formula (2), each of the twenty-four Rs independently represents an alkyl group having from 1 to 3 carbon atoms and, among the twenty-four Rs, two Rs bonded to the same nitrogen atom are optionally bonded to each other.

3. The non-aqueous electrolyte solution for a lithium secondary battery according to claim 1, wherein:
in Formula (1), each of the twenty-four Rs is a methyl group or an ethyl group and, among the twenty-four Rs, two Rs bonded to the same nitrogen atom are optionally bonded to each other, and
in Formula (2), each of the twenty-four Rs is a methyl group or an ethyl group and, among the twenty-four Rs, two Rs bonded to the same nitrogen atom are optionally bonded to each other.

4. The non-aqueous electrolyte solution for a lithium secondary battery according to any one of claims 1 to 3, wherein a content of the phosphazene compound (A) is from 0.1% by mass to 2.0% by mass with respect to a total amount of the non-aqueous electrolyte solution for a battery.

5. The non-aqueous electrolyte solution for a lithium secondary battery according to any one of claims 1 to 4, further comprising a cyclic carbonic acid ester having an unsaturated bond.

6. The non-aqueous electrolyte solution for a lithium secondary battery according to claim 5, wherein the cyclic carbonic acid ester having an unsaturated bond is vinylene carbonate.

7. The non-aqueous electrolyte solution for a lithium secondary battery according to claim 5 or 6, wherein a ratio of a mass content of the cyclic carbonic acid ester having an unsaturated bond with respect to a mass content of the phosphazene compound (A) is in a range of from 0.05 to 30.

8. A lithium secondary battery precursor, comprising:
a casing; and
a positive electrode, a negative electrode, a separator, and an electrolyte solution, which are housed in the casing, wherein:
the positive electrode is configured to occlude, and to release, lithium ions,
the negative electrode is configured to occlude, and to release, lithium ions, and
the electrolyte solution comprises the non-aqueous electrolyte solution for a lithium secondary battery according to any one of claims 1 to 7.

9. The lithium secondary battery precursor according to claim 8, wherein the positive electrode comprises a lithium-containing composite oxide represented by the following general formula (C1) as a positive electrode active material:
LiNiₐCo_{b}Mn_{c}O₂ (C1)
wherein, in the general formula (C1), each of a, b, and c independently represents a number larger than 0 but smaller than 1, and a sum of a, b, and c is from 0.99 to 1.00.

10. A method of manufacturing a lithium secondary battery, the method comprising:
preparing the lithium secondary battery precursor according to claim 8 or 9; and
performing an activation treatment of the lithium secondary battery precursor to obtain a lithium secondary battery,
wherein the activation treatment comprises performing at least one of charging or discharging of the lithium secondary battery precursor in an environment of from 15°C to 70°C.

11. A lithium secondary battery, obtained by charging and discharging the lithium secondary battery precursor according to claim 8 or 9.

12. A phosphazene compound, comprising at least one of a phosphazene compound represented by the following Formula (1) or a phosphazene compound represented by the following Formula (2): 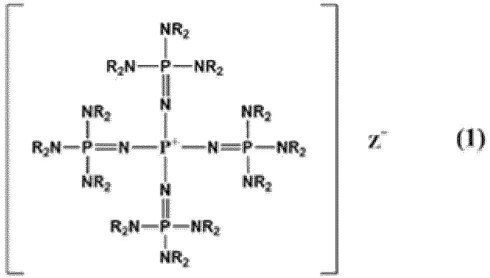 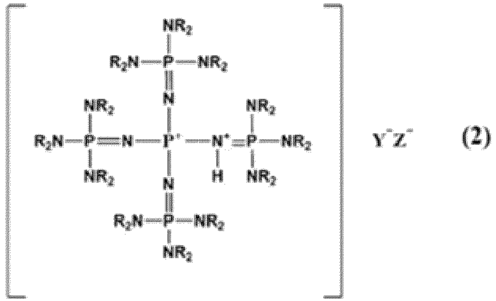
wherein, in Formula (1), Z⁻ represents PO₂F₂⁻; each of twenty-four Rs independently represents a hydrocarbon group having from 1 to 10 carbon atoms; and, among the twenty-four Rs, two Rs bonded to the same nitrogen atom are optionally bonded to each other, and
in Formula (2), each of Y⁻ and Z⁻ independently represents an anion in which a proton is removed from an inorganic acid or an active hydrogen compound; each of twenty-four Rs independently represents a hydrocarbon group having from 1 to 10 carbon atoms; and, among the twenty-four Rs, two Rs bonded to the same nitrogen atom are optionally bonded to each other.

13. A lithium secondary battery additive, comprising a phosphazene compound (A) that comprises at least one of a phosphazene compound represented by the following Formula (1) or a phosphazene compound represented by the following Formula (2): 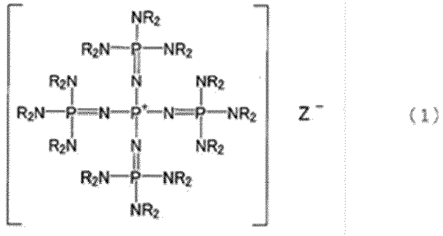 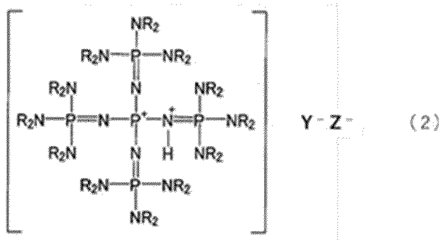
wherein, in Formula (1), Z⁻ represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other, and
in Formula (2), each of Y⁻ and Z⁻ independently represents PF₆⁻, SO₃CF₃⁻, BF₄⁻, ClO₄⁻, PO₂F₂⁻, the following (TFSI⁻), the following (FSI⁻), the following (FOB⁻), the following (BOB⁻), or the following (CA⁻); each of twenty-four Rs independently represents a hydrocarbon group having from 1 to 10 carbon atoms; and, among the twenty-four Rs, two Rs bonded to the same nitrogen atom are optionally bonded to each other

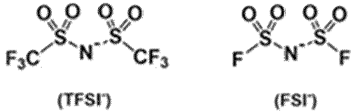

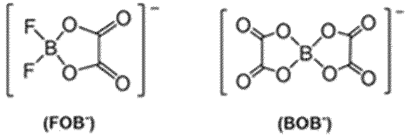

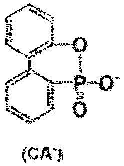

## Patentansprüche

1. Nicht-wässrige Elektrolytlösung für eine Lithium-Batterie, wobei die Lösung eine Phosphazenverbindung (A), die wenigstens eine der Phosphazenverbindung, die durch die folgende Formel (1) dargestellt wird, oder einer Phosphazenverbindung umfasst, die durch die folgende Formel (2) dargestellt wird: 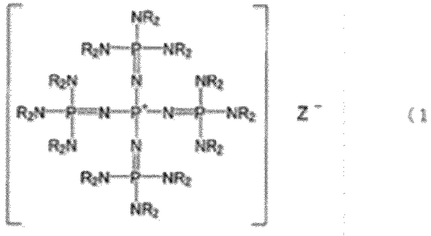 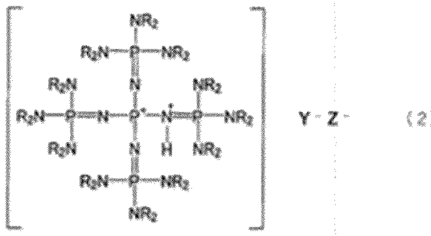
wobei in Formel (1) Z⁻ PF₆⁻, SO₃CF₃⁻, BF₄⁻, ClO₄⁻, PO₂F₂⁻, das folgende (TFSI⁻), das folgende (FSI⁻), das folgende (FOB⁻) das folgende (BOB⁻) oder das folgende (CA⁻) darstellen; jedes der vierundzwanzig R unabhängig eine Kohlenwasserstoffgruppe mit 1 bis 10 Kohlenstoffatomen darstellt; und unter den vierundzwanzig R zwei R, die an dasselbe Stickstoffatom gebunden sind, optional aneinander gebunden sind, und
in Formel (2) jedes von Y⁻ und Z⁻ jeweils unabhängig voneinander PF₆⁻, SO₃CF₃⁻, BF₄⁻ , ClO₄⁻, PO₂F₂⁻, das folgende (TFSI⁻), das folgende (FSI⁻), das folgende (FOB⁻) das folgende (BOB⁻) oder das folgende (CA⁻) darstellen; jedes der vierundzwanzig R unabhängig eine Kohlenwasserstoffgruppe mit 1 bis 10 Kohlenstoffatomen darstellt; und unter den vierundzwanzig R zwei R, die an dasselbe Stickstoffatom gebunden sind, optional aneinander gebunden sind

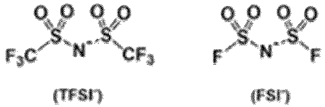

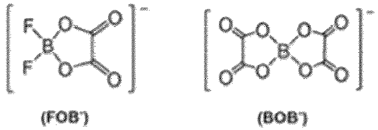

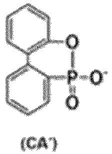

2. Nicht-wässrige Elektrolytlösung für eine Lithium-Batterie nach Anspruch 1, wobei:
in Formel (1) jedes der vierundzwanzig R unabhängig eine Alkylgruppe mit 1 bis 3 Kohlenstoffatomen darstellt und unter den vierundzwanzig R zwei R, die an dasselbe Stickstoffatom gebunden sind, optional aneinander gebunden sind, und
in Formel (2) jedes der vierundzwanzig R unabhängig voneinander eine Alkylgruppe mit 1 bis 3 Kohlenstoffatomen darstellt und unter den vierundzwanzig R zwei R, die an dasselbe Stickstoffatom gebunden sind, optional aneinander gebunden sind.

3. Nicht-wässrige Elektrolytlösung für eine Lithium-Batterie nach Anspruch 1, wobei:
in Formel (1) jedes der vierundzwanzig R unabhängig eine Methylgruppe oder eine Ethylgruppe ist und unter den vierundzwanzig R zwei R, die an dasselbe Stickstoffatom gebunden sind, optional aneinander gebunden sind, und
in Formel (2) jedes der vierundzwanzig R unabhängig voneinander eine Methylgruppe oder eine Ethylgruppe ist und unter den vierundzwanzig R zwei R, die an dasselbe Stickstoffatom gebunden sind, optional aneinander gebunden sind.

4. Nicht-wässrige Elektrolytlösung für eine Lithium-Batterie nach einem der Ansprüche 1 bis 3, wobei ein Gehalt an der Phosphazenverbindung (A) von 0,1 Masse-% bis 2,0 Masse-% in Bezug auf eine Gesamtmenge der nicht-wässrigen Elektrolytlösung für eine Batterie ist.

5. Nicht-wässrige Elektrolytlösung für eine Lithium-Batterie nach einem der Ansprüche 1 bis 4, ferner umfassend einen cyclischen Kohlensäureester mit einer ungesättigten Bindung.

6. Nicht-wässrige Elektrolytlösung für eine Lithium-Batterie nach Anspruch 5, wobei der cyclische Kohlensäureester mit einer ungesättigten Bindung Vinylencarbonat ist.

7. Nicht-wässrige Elektrolytlösung für eine Lithium-Batterie nach Anspruch 5 oder 6, wobei ein Verhältnis des Massengehalts des cyclischen Kohlensäureesters mit einer ungesättigten Bindung in Bezug auf den Massengehalt der Phosphazenverbindung (A) in einem Bereich von 0,05 bis 30 liegt.

8. Lithium-Batterie-Vorläufer, umfassend:
ein Gehäuse; und
eine positive Elektrode, eine negative Elektrode, einen Separator und eine Elektrolytlösung, die in dem Gehäuse untergebracht sind, wobei:
die positive Elektrode dazu konfiguriert ist, Lithium-Ionen einzuschließen und freizusetzen,
die negative Elektrode dazu konfiguriert ist, Lithiumionen einzuschließen und freizusetzen, und
die Elektrolytlösung die nicht-wässrige Elektrolytlösung für eine Lithium-Batterie nach einem der Ansprüche 1 bis 7 umfasst.

9. Lithium-Batterie-Vorläufer nach Anspruch 8, wobei die positive Elektrode ein lithiumhaltiges Mischoxid, das durch die folgende allgemeine Formel (C1) dargestellt ist, als aktives Material für die positive Elektrode umfasst:
LiNiₐCo_{b}Mn_{c}O₂ (C1)
wobei in der allgemeinen Formel (C1) a, b und c jeweils unabhängig voneinander eine Zahl größer als 0, aber kleiner als 1 darstellt, und die Summe von a, b und c von 0,99 bis 1,00 beträgt.

10. Verfahren zum Fertigen einer Lithium-Batterie, wobei das Verfahren umfasst:
Herstellen des Lithium-Batterie-Vorläufers nach Anspruch 8 oder 9;
Durchführen einer Aktivierungsbehandlung des Lithium-Batterie-Vorläufers, um eine Lithium-Batterie zu erhalten,
wobei die Aktivierungsbehandlung das Durchführen von wenigstens einem von Laden oder Entladen des Lithium-Batterie-Vorläufers in einer Umgebung von 15 °C bis 70 °C umfasst.

11. Lithium-Batterie, erhalten durch Laden und Entladen des Lithium-Batterie-Vorläufers nach Anspruch 8 oder 9.

12. Phosphazenverbindung, umfassend wenigstens eine einer Phosphazenverbindung, die durch die folgende Formel (1) dargestellt wird, oder einer Phosphazenverbindung, die durch die folgende Formel (2) dargestellt wird: 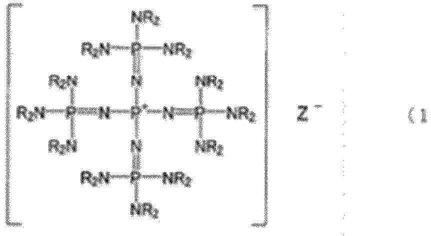 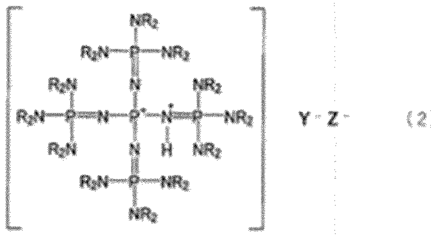
wobei in Formel (1) Z⁻ PO₂F₂⁻ darstellt; jedes der vierundzwanzig R unabhängig eine Kohlenwasserstoffgruppe mit 1 bis 10 Kohlenstoffatomen darstellt; und unter den vierundzwanzig R zwei R, die an dasselbe Stickstoffatom gebunden sind, optional aneinander gebunden sind, und
in Formel (2) jedes von Y⁻ und Z⁻ jeweils unabhängig voneinander ein Anion darstellen, in dem ein Proton von einer anorganischen Säure oder einer aktiven Kohlenwasserstoffverbindung entfernt wurde; jedes der vierundzwanzig R unabhängig eine Kohlenwasserstoffgruppe mit 1 bis 10 Kohlenstoffatomen darstellt; und unter den vierundzwanzig R zwei R, die an dasselbe Stickstoffatom gebunden sind, optional aneinander gebunden sind.

13. Lithium-Batteriezusatzstoff, umfassend wenigstens eine Phosphazenverbindung (A), die wenigstens eine einer Phosphazenverbindung, die durch die folgende Formel (1) dargestellt wird, oder einer Phosphazenverbindung umfasst, die durch die folgende Formel (2) dargestellt wird: 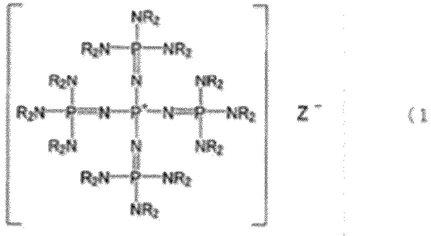 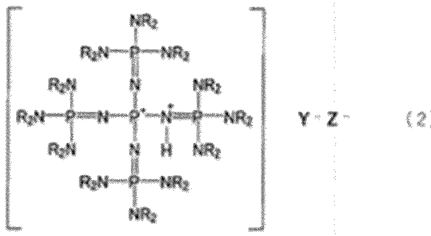
wobei in Formel (1) Z⁻ PF₆⁻, SO₃CF₃⁻, BF₄⁻, ClO₄⁻, PO₂F₂⁻, das folgende (TFSI⁻), das folgende (FSI⁻), das folgende (FOB⁻) das folgende (BOB⁻) oder das folgende (CA⁻) darstellt; jedes der vierundzwanzig R unabhängig eine Kohlenwasserstoffgruppe mit 1 bis 10 Kohlenstoffatomen darstellt; und unter den vierundzwanzig R zwei R, die an dasselbe Stickstoffatom gebunden sind, optional aneinander gebunden sind, und
in Formel (2) jedes von Y⁻ und Z⁻ jeweils unabhängig voneinander PF₆⁻, SO₃CF₃⁻, BF₄⁻ , ClO₄⁻, PO₂F₂⁻, das folgende (TFSI⁻), das folgende (FSI⁻), das folgende (FOB⁻) das folgende (BOB⁻) oder das folgende (CA⁻) darstellen; jedes der vierundzwanzig R unabhängig eine Kohlenwasserstoffgruppe mit 1 bis 10 Kohlenstoffatomen darstellt; und unter den vierundzwanzig R zwei R, die an dasselbe Stickstoffatom gebunden sind, optional aneinander gebunden sind

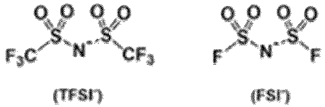

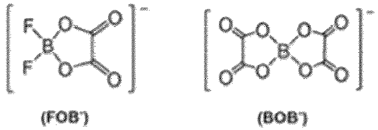

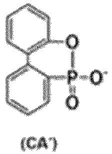

## Revendications

1. Solution électrolytique non aqueuse pour une batterie secondaire au lithium, cette solution comprenant un composé phosphazène (A) qui comprend au moins l'un parmi un composé phosphazène représenté par la formule (1) suivante ou un composé phosphazène représenté par la formule (2) suivante : 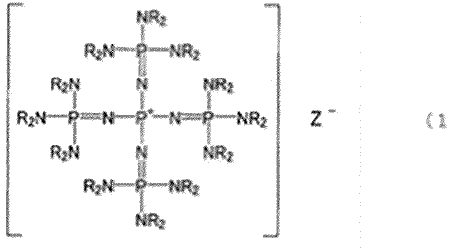 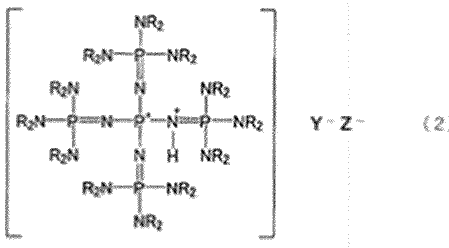 dans laquelle, dans la formule (1), Z⁻ représente PF₆⁻, SO₃CF₃⁻, BF₄⁻, ClO₄⁻, PO₂F₂⁻, le (TFSI⁻) suivant, le (FSI⁻) suivant, le (FOB⁻) suivant, le (BOB⁻) suivant, ou le (CA⁻) suivant ; chacun de vingt-quatre R représente indépendamment un groupe hydrocarboné ayant 1 à 10 atomes de carbone ; et, parmi les vingt-quatre R, deux R liés au même atome d'azote sont éventuellement liés l'un à l'autre, et
dans la formule (2), Y⁻ et Z⁻ représentent chacun indépendamment PF₆⁻, SO₃CF₃⁻, BF₄⁻, ClO₄⁻, PO₂F₂⁻, le (TFSI⁻) suivant, le (FSI⁻) suivant, le (FOB⁻) suivant, le (BOB⁻) suivant, ou le (CA⁻) suivant ; chacun de vingt-quatre R représente indépendamment un groupe hydrocarboné ayant 1 à 10 atomes de carbone ; et, parmi les vingt-quatre R, deux R liés au même atome d'azote sont éventuellement liés l'un à l'autre,

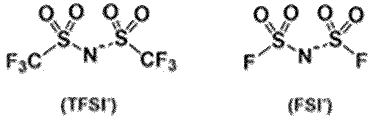

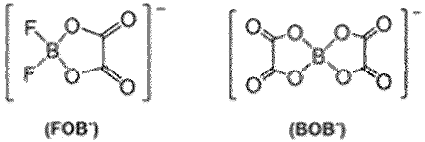

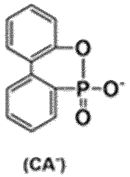

2. Solution électrolytique non aqueuse pour une batterie secondaire au lithium selon la revendication 1, dans laquelle :
dans la formule (1), chacun des vingt-quatre R représente indépendamment un groupe alkyle ayant 1 à 3 atomes de carbone et, parmi les vingt-quatre R, deux R liés au même atome d'azote sont éventuellement liés l'un à l'autre, et
dans la formule (2), chacun des vingt-quatre R représente un groupe alkyle ayant 1 à 3 atomes de carbone et, parmi les vingt-quatre R, deux R liés au même atome d'azote sont éventuellement liés l'un à l'autre.

3. Solution électrolytique non aqueuse pour une batterie secondaire au lithium selon la revendication 1, dans laquelle :
dans la formule (1), chacun des vingt-quatre R est un groupe méthyle ou un groupe éthyle et, parmi les vingt-quatre R, deux R liés au même atome d'azote sont éventuellement liés l'un à l'autre, et
dans la formule (2), chacun des vingt-quatre R est un groupe méthyle ou un groupe éthyle et, parmi les vingt-quatre R, deux R liés au même atome d'azote sont éventuellement liés l'un à l'autre,

4. Solution électrolytique non aqueuse pour une batterie secondaire au lithium selon l'une quelconque des revendications 1 à 3, dans laquelle la teneur en composé phosphazène (A) est de 0,1 % à 2,0 % en masse par rapport à une quantité totale de la solution électrolytique non aqueuse pour une batterie.

5. Solution électrolytique non aqueuse pour une batterie secondaire au lithium selon l'une quelconque des revendications 1 à 4, comprenant en outre un ester d'acide carbonique cyclique ayant une liaison insaturée.

6. Solution électrolytique non aqueuse pour une batterie secondaire au lithium selon la revendication 5, dans laquelle l'ester d'acide carbonique cyclique ayant une liaison insaturée est le carbonate de vinylène.

7. Solution électrolytique non aqueuse pour une batterie secondaire au lithium selon la revendication 5 ou 6, dans laquelle un rapport d'une teneur massique en ester d'acide carbonique cyclique ayant une liaison insaturée par rapport à une teneur massique en composé phosphazène (A) est dans une plage de 0,05 à 30.

8. Précurseur de batterie secondaire au lithium, comprenant :
un boîtier ; et
une électrode positive, une électrode négative, un séparateur, et une solution électrolytique, qui sont logés dans le boîtier, dans lequel :
l'électrode positive est configurée pour occlure, et pour libérer, des ions lithium,
l'électrode négative est configurée pour occlure, et pour libérer, des ions lithium, et
la solution électrolytique comprend la solution électrolytique non aqueuse pour une batterie secondaire au lithium selon l'une quelconque des revendications 1 à 7.

9. Précurseur de batterie secondaire au lithium selon la revendication 8, dans lequel l'électrode positive comprend un oxyde composite contenant du lithium représenté par la formule générale (C1) suivante en tant que matériau actif d'électrode positive :
LiNiₐCo_{b}Mn_{c}O₂ (C1)
dans lequel, dans la formule générale (C1), a, b et c représentent chacun indépendamment un nombre supérieur à 0 mais inférieur à 1, et une somme de a, b, et c est de 0,99 à 1,00.

10. Procédé de fabrication d'une batterie secondaire au lithium, le procédé comprenant :
la préparation du précurseur de batterie secondaire au lithium selon la revendication 8 ou 9 ; et
la réalisation d'un traitement d'activation du précurseur de batterie secondaire au lithium pour obtenir une batterie secondaire au lithium,
dans lequel le traitement d'activation comprend la réalisation d'au moins l'une parmi la charge ou la décharge du précurseur de batterie secondaire au lithium dans un environnement compris de 15 °C à 70 °C.

11. Batterie secondaire au lithium, obtenue par la charge et la décharge du précurseur de batterie secondaire au lithium selon la revendication 8 ou 9.

12. Composé phosphazène, comprenant au moins l'un parmi un composé phosphazène représenté par la formule (1) suivante ou un composé phosphazène représenté par la formule (2) suivante : 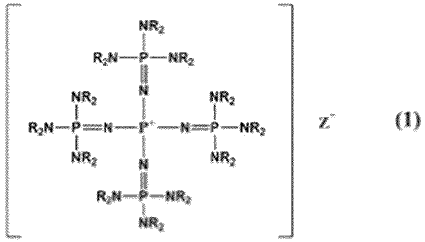 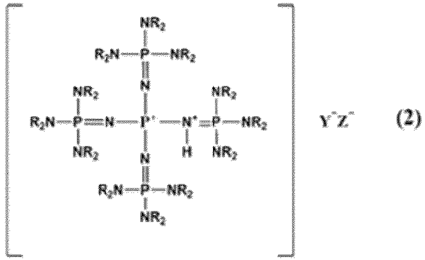
dans lequel, dans la formule (1), Z⁻ représente PO₂F₂⁻ ; chacun de vingt-quatre R représente indépendamment un groupe hydrocarboné ayant 1 à 10 atomes de carbone ; et, parmi les vingt-quatre R, deux R liés au même atome d'azote sont éventuellement liés l'un à l'autre, et
dans la formule (2), Y⁻ et Z⁻ représentent chacun un anion dans lequel un proton est retiré d'un acide inorganique ou d'un composé hydrogène actif ; chacun de vingt-quatre représente indépendamment un groupe hydrocarboné ayant 1 à 10 atomes de carbone ; et, parmi les vingt-quatre R, deux R liés au même atome d'azote sont éventuellement liés l'un à l'autre.

13. Additif de batterie secondaire au lithium, comprenant un composé phosphazène (A) qui comprend au moins l'un parmi un composé phosphazène représenté par la formule (1) suivante ou un composé phosphazène représenté par la formule (2) suivante : 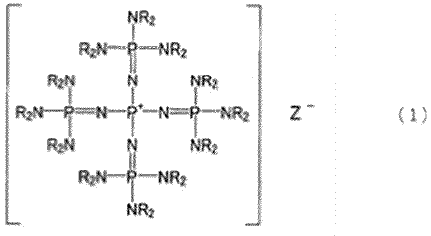 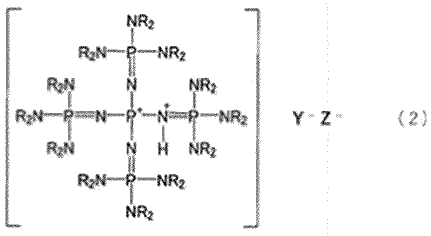
dans lequel, dans la formule (1), Z⁻ représente PF₆⁻, SO₃CF₃⁻, BF₄⁻, ClO₄⁻, PO₂F₂⁻, le (TFSI⁻) suivant, le (FSI⁻) suivant, le (FOB⁻) suivant, le (BOB⁻) suivant, ou le (CA⁻) suivant ; chacun de vingt-quatre R représente indépendamment un groupe hydrocarboné ayant 1 à 10 atomes de carbone ; et, parmi les vingt-quatre R, deux R liés au même atome d'azote sont éventuellement liés l'un à l'autre, et
dans la formule (2), Y⁻ et Z⁻ représentent chacun indépendamment PF₆⁻, SO₃CF₃⁻, BF₄⁻, ClO₄⁻, PO₂F₂⁻, le (TFSI⁻) suivant, le (FSI⁻) suivant, le (FOB⁻) suivant, le (BOB⁻) suivant, ou le (CA⁻) suivant ; chacun de vingt-quatre R représente indépendamment un groupe hydrocarboné ayant 1 à 10 atomes de carbone ; et, parmi les vingt-quatre R, deux R liés au même atome d'azote sont éventuellement liés l'un à l'autre
